# EUROPÄISCHE PATENTSCHRIFT

(11) **EP 2 451 775 B1**
(45) Veröffentlichungstag und Bekanntmachung des Hinweises auf die Patenterteilung: **30.05.2018**
(21) Anmeldenummer: 10730368.7
(22) Anmeldetag: 26.06.2010
(51) Int. Cl.: C01B 32/80, C07C 263/10, C07C 265/14

(54) **VERFAHREN ZUR HERSTELLUNG VON ISOCYANATEN**
METHOD FOR PRODUCING ISOCYANATES
PROCÉDÉ POUR PRÉPARER DES ISOCYANATES

(30) Priorität: 09.07.2009 DE 102009032413
(43) Veröffentlichungstag der Anmeldung: 16.05.2012
(73) Patentinhaber: Covestro Deutschland AG, 51373 Leverkusen (DE)
(72) Erfinder: BRUNS, Rainer, 51373 Leverkusen (DE); LORENZ, Wolfgang, 41540 Dormagen (DE); STEFFENS, Friedhelm, 51373 Leverkusen (DE); STUTZ, Herbert, 41541 Dormagen (DE)
(74) Vertreter: Levpat
(86) Internationale Anmeldenummer: PCT/EP2010/003916
(87) Internationale Veröffentlichungsnummer: WO 2011/003532

(56) Entgegenhaltungen:
- EP-A1- 1 849 767
- WO-A1-2008/086922
- WO-A1-2009/037179

## Beschreibung

Die Erfindung betrifft ein Verfahren zur Herstellung von Isocyanaten durch Umsetzung von primären Aminen mit Phosgen im stöchiometrischen Überschuss in der Gasphase, bei dem das überschüssige Phosgen anschließend zurückgewonnen und in die Umsetzung zurückgeführt wird.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen. Bei diesen Synthesen fällt das überschüssige Phosgen in der Regel zumindest teilweise zusammen mit dem in der Reaktion freigesetzten gasförmigen Nebenprodukt Chlorwasserstoff an, so dass es für einen wirtschaftlichen Betrieb einer Isocyanatsynthese unabdingbar ist, das überschüssige Phosgen vom Nebenprodukt Chlorwasserstoff abzutrennen und in die Reaktion zurückzuführen.

Die vorliegende Erfindung betrifft insbesondere ein Verfahren zur Rückgewinnung von Phosgen-überschüssen wie sie bei der Herstellung von Isocyanaten aus Aminen und Phosgen in der Gasphasenphosgenierung anfallen, und zur Rückführung des zurückgewonnenen Phosgens zum Gasphasenreaktor.

Es sind aus dem Stand der Technik verschiedene Verfahren zur Herstellung von Isocyanaten durch Umsetzung von Aminen mit Phosgen in der Gasphase bekannt.

EP-A-289 840 beschreibt die Herstellung von aliphatischen Diisocyanaten durch Gasphasenphosgenierung, wobei Phosgen im Überschuss eingesetzt wird. Die Schrift beschreibt, dass der die Reaktion verlassende Gasstrom in an sich bekannter Weise von überschüssigem Phosgen befreit werden kann, wobei eine Kühlfälle oder Absorption in einem Lösemittel bei einer Temperatur von - 10°C bis 8°C oder eine Adsorption und Hydrolyse an Aktivkohle erwähnt wird. In Beispiel 1 wird der die Reaktion verlassende Gasstrom durch Adsorption in einem Aktivkohleturm von Phosgen befreit. Die Schrift lehrt, dass durch Anlegen eines Differenzdruckes zwischen den Zuleitungen zum Reaktionsraum und dem Ausgang aus dem Reaktionsraum die Strömungsgeschwindigkeit in dem Reaktionsraum eingestellt werden kann, offenbart jedoch nicht wie der Differenzdruck erzeugt wird. Insbesondere nachteilig an dem offenbarten Verfahren ist, dass bei einer derartigen Aufarbeitung das überschüssige Phosgen vernichtet wird und nicht mehr in der Reaktion eingesetzt werden kann und somit ein wirtschaftlicher Betrieb nicht gegeben ist.

EP-A-570 799 betrifft ein Verfahren zur Herstellung von aromatischen Diisocyanaten, dadurch gekennzeichnet, dass die Umsetzung des zugehörigen Diamins mit dem Phosgen im Überschuss in einem Rohrreaktor oberhalb der Siedetemperatur des Diamins innerhalb einer mittleren Kontaktzeit von 0,5 bis 5 Sekunden durchgeführt wird. Die Schrift beschreibt, dass das die Kondensationsstufe des Reaktors verlassende Gasgemisch in an sich bekannter Weise von Phosgen befreit werden kann. Hierbei wird eine Kühlfalle, eine Absorption in einem kalten Lösemittel sowie die Adsorption und Hydrolyse an Aktivkohle erwähnt. Das Beispiel beschreibt die Hydrolyse des überschüssigen Phosgens mit Wasser. Diese Vernichtung des überschüssigen Phosgens ist in Hinsicht auf eine wirtschaftliche Verfahrensführung nachteilig.

GB-A-1 165 831 beschreibt ein Verfahren zur Herstellung von Isocyanaten in der Gasphase, bei dem die Umsetzung des dampfförmigen Amins mit dem Phosgen bei Temperaturen zwischen 150°C und 300°C in einem mit einem mechanischen Rührer ausgestatteten und über einen Heizmantel temperierbaren Rohrreaktor durchgeführt wird. Nachteilig an dem offenbarten Verfahren ist zum einen die Verwendung eines schnell laufenden Rührers und dessen externer Antrieb über eine die Reaktorwandung durchdringende Welle, da ein solches Rührwerk beim Einsatz von Phosgen einen sehr hohen Aufwand zur sicherheitstechnisch notwendigen Abdichtung erfordert. Im Beispiel wird beschrieben, dass die den Reaktor verlassenden Dämpfe durch Absorption in kaltem Monochlorbenzol absorbiert werden. Es wird keine Anleitung gegeben, ob und auf welche Art das im Lösemittel absorbierte Phosgen in der Reaktion wieder verwendet werden kann.

GB 737 442 beschreibt ein Verfahren zur Rückgewinnung von flüssigem Phosgen aus HCl und Phosgen enthaltenden Gasmischungen, dadurch das das Gasgemisch aufwärts durch einen auf -40 bis -60°C gekühlten Kühler strömt, wobei das Phosgen kondensiert und in einen Vorratstank abläuft. Diese Schrift offenbart nicht, wie das zurückgewonnene flüssige Phosgen in einer Gasphasenreaktion verwendet werden kann. Nachteilig an dem offenbarten Verfahren ist, dass das den Kühler verlassende HCl Gas noch nennenswerte Mengen Phosgen enthält, die somit für die Phosgenierreaktion verloren sind. Weiterhin nachteilig ist das die Kondensation auf sehr niedrigem und damit energetisch aufwendigem Temperaturniveau durchgeführt wird.

US 2 764 607 beschreibt ein Verfahren zur Rückgewinnung von Phosgen aus einer Gasmischung mit HCl aus der Produktion von Chloroformaten. Dazu wird das den Kondensator, der auf den Reaktionskessel montiert ist, verlassene Gas zunächst in Kontakt mit kaltem Lösemittel gebracht, wobei das Phosgen in dem Lösemittel absorbiert wird. Daran schließt sich ein Extraktionsschritt an, in dem das Phosgen zusammen mit dem partiell mit absorbierten HCl kontinuierlich in einer Destillationskolonne von dem Lösemittel getrennt wird. Daran schließt sich die Reinigung des erhaltenden Gasstromes durch Kondensation des Lösemittels und daran die Verflüssigung des Phosgens an, das einem flüssigen Lagerbehälter zugeführt wird. Die Schrift offenbart keine Lehre, welche Druckverhältnisse zwischen dem Absorptionsschritt und dem Extraktionsschritt herrschen. Nachteilig an dem offenbarten Verfahren ist, dass die Lagerung von flüssigem Phosgen mit einem hohen Gefahrenpotential einhergeht.

Nach der Lehre von EP 1 849 767 erfordert ein ökonomischer Isocyanatproduktionsprozess die Abtrennung und Rückgewinnung des überschüssigen Phosgens von dem Koppelprodukt HCl sowie die Gewinnung der HCl in ausreichender Reinheit, so dass sie in weiteren Synthesen und Anwendungsgebieten ohne weitere Behandlung verwendet werden kann. Dazu offenbart die Schrift ein Verfahren, in dem das Gasgemisch in einem Lösemittel zweistufig absorbiert wird, wobei der erste Schritt isotherm und der zweite Schritt adiabatisch durchgeführt wird. Dabei wird eine Lösung von Phosgen im Absorptionsmittel sowie ein HCl Gas in gewünschter Reinheit erhalten. Die Schrift beschreibt, dass für Isocyanatproduktionen nach dem Gasphasenverfahren die erhaltene Phosgenlösung in einem folgenden Schritt desorbiert werden kann, wobei nach Lehre von EP 1 849 767 die Absorption bevorzugt bei tiefen Temperaturen und hohen Drücken und die Desorption bevorzugt bei hohen Temperaturen und niedrigen Drücken durchgeführt wird.

Ein Alternatives Verfahren zur Auftrennung von gasförmigen Gemischen aus Chlorwasserstoff und Phosgen beschreibt DE 102 600 84**.** Die Schrift offenbart ein Verfahren, bei dem das Phosgen unter erhöhtem Druck kondensiert und die kondensierte Phase in einem anschließenden Verfahrensschritt zur Entfernung des Chlorwasserstoffes aus dem Sumpfprodukt Phosgen, d.h. in der flüssigen Phase, gestrippt wird. Die Strippung ist notwendig, da sich aufgrund des erhöhten Druckes nennenswerte Mengen HCl in dem Kondensat lösen und diese nach Lehre der Schrift in Phosgenierreaktionen sich nachteilig auswirken. Nachteilig an dem offenbarten Verfahren ist, dass aufgrund des herrschenden Kondensationsdruckes ein weiterer Verfahrensschritt zum Abtrennung der gelösten HCl notwendig ist. Das Dokument liefert keine Anleitung zur Rückgewinnung von gasförmigem Phosgen. Die Schrift beschreibt, dass die HCl- Phosgen-Trennung unter hohem Druck durchgeführt werden kann, dieses jedoch das Sicherheitsrisiko erhöht. Zudem ist die Erzeugung von hohem Druck energetisch aufwendig. Als Alternative wird die Trennung bei sehr niedrigen Temperaturen beschrieben, die jedoch ebenfalls energetisch aufwendig ist und zudem zu hohen Gehalten an HCl in der flüssigen Phosgen enthaltenden Phase führt.

WO 2007 014 936 offenbart ein Verfahren zur Herstellung von Diisocyanaten durch Umsetzung von Diaminen mit Phosgen im stöchiometrischen Überschuss, wobei das überschüssige Phosgen zumindest teilweise wieder in die Reaktion geführt wird und wobei der Phosgenstrom zum Reaktor vor der Vermischung mit dem Amin weniger als 15 Gew% HCl enthält. Damit soll nach der Lehre dieser Schrift die Standzeit der Reaktoren verbessert werden, da Ausfällungen von Aminhydrochloriden verringert werden sollen. Nachteilig an solch hohen Gehalten an inertem HCl Gas in dem Phosgengas ist, dass dieses zu großen Apparaten und damit zu hohen Anlagenbaukosten führt.

Darüber hinaus erhöht das inerte HCl Gas im Phosgengas die Kreislaufströme, was zu einer Erhöhung der Betriebskosten führt. Somit ist es generell stets erstrebenswert die Inertgasbelastung der Verfahren zu minimieren. Als Ausführungsform wird beschrieben, dass das überschüssige Phosgen und der entstandene Chlorwasserstoff zunächst von dem im wesentlichen gasförmigen Reaktionsgemisch abgetrennt wird, dann das überschüssige Phosgen zumindest teilweise wieder in die Reaktion zurückgeführt wird, wobei aus diesem rückgeführten Phosgen Chlorwasserstoff so abgetrennt wird, dass der Phosgenstrom vor der Vermischung mit dem Aminstrom weniger als 15 Gew% HCl enthält. Die Schrift beschreibt, dass die Trennung bevorzugt über eine Kombination von einer Destillation und einer Wäsche durchgeführt wird. Dabei wird das Phosgen aus dem Chlorwasserstoff haltigen Strom mit einem Waschmittel ausgewaschen. Die Abtrennung des Phosgens und des Chlorwasserstoffes aus diesem beladenen Waschmedium erfolgt bevorzugt destillativ. Die Wäsche und die Destillation können nach Beschreibung bei Drücken von 1 bis 10 bar absolut betrieben werden. Die Schrift offenbart nicht die relativen Druckverhältnisse zwischen der Wäsche und der Destillation.

Nach der Lehre von WO 2008 086 922 darf in einer Gasphasenphosgenierreaktion das Phosgen vor der Vermischung mit dem Amin nicht mehr als 1000 Gew-ppm Chlor enthalten, da sonst aufgrund der hohen Temperaturen die Gefahr von Werkstoffversprödungen eintreten würde. Nach dieser Lehre bildet sich aufgrund der Spaltung von Phosgen bei hohen Temperaturen stets eine gewisse Menge Chlor, so dass eine Abtrennung dieses Chlors notwendig ist. Dazu offenbart die Schrift eine Ausführung, in der das Phosgen, HCl und Chlor enthaltende Gasgemisch zunächst einer partiellen Kondensation (S. 18, Z.30) und Wäsche (S. 19, Z.18) jeweils bei einem Druck von 0.1 bis 20 bar absolut unterworfen wird. Dabei wird eine flüssige Phase enthaltend Phosgen, Waschmedium, HCl und Chlor erhalten. Diese wird dann in einer Rektifikation von den Leichtsiedern Chlor und HCl bei einem Druck von 1 bis 5 bar absolut befreit. In einem daran anschließenden Schritt wird in einer Rektifikation bei einem Druck von 1 bis 5 bar absolut (S.21, Z. 2) Phosgen und Waschmedium voneinander getrennt, wobei ein Phosgenstrom in gewünschter Chlorreinheit erhalten wird der wieder in der Phosgenierung eingesetzt werden kann. Nach allgemeiner Lehre dieser Schrift ist es also vorteilhaft die partielle Kondensation und Wäsche unter einem höheren Druck als die Rektifikationsschritte zu betreiben. Weiterhin ist nach Lehre dieser Schrift ein zweistufiges Destillationsverfahren notwendig, um gasförmiges Phosgen in ausreichender Reinheit für eine Phosgenierreaktion aus einem mit Phosgen beladenen Waschmedium zu erhalten.

WO 2009 037 179 offenbart ein Verfahren zur Herstellung von Isocyanaten in der Gasphase, wobei das Phosgen in allen Verfahrensschritten im wesentlichen gasförmig vorliegt und somit auf die Zuführung von Energie zur Verdampfung von flüssigem Phosgen verzichtet werden kann. Dieses wird nach Lehre der Schrift erreicht, in dem das in der Phosgenerzeugung erhaltene gasförmige Phosgen insbesondere ohne Zwischenkondensation in die Gasphasenphosgenierung eingebracht wird.

Weiterhin beschreibt diese Schrift ein Verfahren zur Abtrennung von Phosgen aus einem Gasgemisch mit HCl und Rückführung des abgetrennten Phosgens zur Gasphasenphosgenierung durch eine kombinierte Wäsche und mehrstufige Destillation, die unter einem Druck von 1 bis 10 bar absolut betrieben wird. Die Schrift offenbart nicht die relativen Druckverhältnisse zwischen der Wäsche und der Destillation.

Die Schrift führt aus, dass zunächst in einem ersten Schritt durch Wäsche des Phosgen-HCl-Gasgemisches mit einer Waschflüssigkeit eine mit Phosgen und HCl beladene Waschflüssigkeit erhalten wird. Daran schließt sich ein erster Destillationsschritt an, in dem das HCl weitestgehend aus der phosgenhaltigen Waschlösung entfernt und wieder in den vorgelagerten Waschschritt zurückgeführt wird, gefolgt von einem zweiten Destillationsschritt, in dem die zuvor erhaltende Waschlösung in gasförmiges Phosgen und weitestgehend phosgenfreie Waschflüssigkeit aufgetrennt wird. Das gasförmige Phosgen wird in die Gasphasenphosgenierung geleitet, während die Waschflüssigkeit erneut zur Wäsche des Phosgen-HCl-Gasgemisches verwendet wird. Nach allgemeiner Lehre dieser Schrift ist folglich ein zweistufiges Destillationsverfahren mit HCl Rückführung zur vorgeschalteten Wäsche notwendig, um Phosgen aus Phosgen- und HCl-enthaltenden Gasgemischen zurückzugewinnen und für eine Gasphasenphosgenierung verwenden zu können.

Die Anmeldung WO 2009/037179 A1 betrifft ein Verfahren zur Herstellung von Diisocyanaten aus Diaminen und Phosgen in der Gasphase, bei dem das in den Verfahrensstufen befindliche Phosgen im Wesentlichen gasförmig vorliegt. Es wird eine Ausführungsform offenbart, in welcher die im Wesentlichen Phosgen und Chlorwasserstoff sowie gegebenenfalls Lösungsmittel enthaltenden, vereinigten gasförmigen Ströme aus der Aufarbeitungsstufe (VII) und/oder aus der Reinigungsstufe (VIII) bei einem Druck von bevorzugt 1 bis 5 bar in einer Waschvorrichtung (IXa) mit Waschflüssigkeit (21) beaufschlagt werden, wobei ein Chlorwasserstoffstrom (20) und ein Strom 22 aus mit Phosgen und gegebenenfalls Chlorwasserstoff beladener Waschflüssigkeit erhalten werden. Strom 22 wird in eine Trennblechdestillation (Stufe (IXd)) geleitet. In der Stufe (IXd) wird aus Strom 22 ein Phosgenstrom 19 gewonnen, welcher in die Reaktion zurückgeführt wird. Ferner wird dort ein zweiter Chlorwasserstoffstrom (20) gewonnen. Die Trennblechkolonne wird dabei in der Regel bei einem Druck von 0,5 - 5 bar über dem in der Wascheinheit (IXa) betrieben. Die Anmeldung geht auf die Vor- und Nachteile dieser Art der Druckführung nicht ein.

EP 1 849 767 A1 beschreibt ein Verfahren zur Herstellung von Isocyanaten, bei dem der in der Phosgenierungsreaktion erhaltene Gasstrom enthaltend Chlorwasserstoff, Phosgen und ggf. Lösungsmittel, Leichtsieder sowie Inerte in eine zweistufige Absorption umfassend einen isotherm und einen adiabatisch geführten Absorptionsschritt unter Erhalt von (i) eines Chlorwasserstoffstroms und (ii) eines flüssigen Phosgenstroms geführt wird.

Überraschenderweise wurde nun gefunden, dass sich zur Rückgewinnung von Phosgen aus Gasgemischen enthaltend Phosgen und HCl, wie sie durch die Umsetzung von Phosgen im stöchiometrischen Überschuss mit primären Aminen, in einem Reaktor entstehen, und der sich anschließenden Rückführung des zurückgewonnenen Phosgens zu dem Reaktor insbesondere eine Sequenz aus zwei Verfahrensschritten eignet. Dabei wird im ersten Schritt (HCl-Phosgen-Trennung) das HCl und Phosgen enthaltende Gasgemisch, das den Reaktor verlässt, in einen gasförmigen im wesentlichen HCl enthaltenden Strom und einen flüssigen Phosgen enthaltenden Strom aufgetrennt, und in einem zweiten Schritt (Phosgengaserzeugung) der zuvor erhaltende flüssige Strom zumindest teilweise in einen gasförmigen Phosgen enthaltenden Strom überführt, wobei der Druck im ersten Verfahrens-Schritt geringer ist als der Druck im zweiten Verfahrens-Schritt. Der Einsatz des erfindungsgemäßen Verfahrens mit den erfindungsgemäßen Verfahrensbedingungen ermöglicht den Verzicht auf Druckerhöhungselemente im Gasweg des zum Reaktor zurückgeführten Phosgens. Dadurch wird die Sicherheit der Produktionsanlage erhöht. Insbesondere wird dadurch der Verzicht von Druckerhöhungselementen im gesamten Phosgengasraum erreicht. Insbesondere ist es durch das erfindungsgemäße Verfahren möglich gasförmiges Phosgen aus Phosgen und HCl enthaltenden Gasgemischen zurückzugewinnen, ohne dass dabei ein Gasstrom aus dem zweiten Verfahrenschritt der Phosgengaserzeugung in den ersten Verfahrensschritt der HCl-Phosgen-Trennung zurückgeführt werden muss. Dadurch wird die Anzahl der Apparate verringert und der Energieaufwand des Prozesses verringert.

Durch das erfindungsgemäße Verfahren in Kombination mit den darin geforderten Verfahrensbedingungen kann eine hohe Phosgenrückgewinnungsausbeute erreicht werden, d.h. ein hoher Anteil des Phosgens aus dem Gasgemisch enthaltend Phosgen und HCl wird abgetrennt und der Reaktion wieder zugeführt. Dadurch werden die Phosgenverluste minimiert und die Wirtschaftlichkeit des Verfahrens verbessert.

Der Einsatz der geforderten Verfahrenssequenz in Kombination mit den geforderten Betriebsbedingungen in dem erfindungsgemäßen Verfahren ist insofern besonders überraschend, als nach Lehre des Stands der Technik die Überführung von Phosgen aus der flüssigen Phase in die gasförmige Phase energetisch vorteilhaft bei einem geringerem Druck betrieben wird als die vorgelagerte Abtrennung des Phosgens aus dem HCl-Phosgen-Gasgemisch wie z.B. durch Absorption von Phosgen in einem Absorptionsmittel und anschließende Desorption.

Insofern war es nach Lage des Standes der Technik für den Fachmann nicht zu erwarten, dass eine bzgl des Druckes umgekehrte Prozessführung zwischen den Verfahrensschritten HCl-Phosgen-Trennung und Phosgengaserzeugung zu einem energetisch vorteilhaften Gesamtverfahren führt.

Weiterhin wurde überraschenderweise gefunden, dass der unter einem geringeren Druck als die Phosgengaserzeugung betriebene erste Verfahrensschritt der HCl-Phosgen-Trennung trotz des geringen Druckes ein für die weitere Verarbeitung ausreichend reines HCl Gas erzeugt. Dieses ist überraschend da nach Lehre des Standes der Technik die Reinheit des im ersten Verfahrensschritt erzeugten HCl Gases mit dem in diesem Schritt herrschenden Druck steigt.

Insbesondere ist es für den Fachmann überraschend, das trotz des geringen Druckes in dem ersten Verfahrenschritt der HCl-Phosgen-Trennung das Phosgen weitestgehend aus dem HCl und Phosgen enthaltenden Gasgemisch abgetrennt werden kann und somit die Phosgenverluste in dieser Verfahrenstufe gering sind. Dieses ermöglicht in Kombination mit dem Verzicht einer gasförmigen Rückführung aus dem zweiten Verfahrensschritt der Phosgengaserzeugung in den ersten Verfahrensschritt der HCl-Phosgen-Trennung eine hohe Phosgenrückgewinnungsausbeute.

Der Einsatz der im erfindungsgemäßen Verfahren vorgesehenen Verfahrens-Sequenz ist vorteilhaft, da der im ersten Schritt der HCl-Phosgen-Trennung erzeugte Strom nur geringe Mengen gelöste HCl und gelöste inerte Gase enthält. Dieses ist insbesondere vorteilhaft, da dadurch die Gasbelastung der Apparate in den Verfahrenschritten Phosgengaserzeugung und Reaktion durch Inerte geringer ist und somit diese Apparate kleiner gebaut werden können. Es ist zudem auch vorteilhaft, da durch die geringe Menge gelöster HCl der energetische Aufwand zur Erzeugung des Phosgengases im folgenden Verfahrensschritt verringert wird. Weiterhin ist es dadurch möglich auf die nach dem Stand der Technik notwendige zweistufige Destillation mit Rückführung eines gasförmigen HCl Stromes zur HCl-Phosgen-Trennung zu verzichten, so dass aus dem zweiten Verfahrensschritt der Phosgengaserzeugung kein gasförmiger Strom in den ersten Verfahrenschritt der HCl-Phosgentrennung zurückgeführt werden muss. Durch geeignete Wahl der Verfahrensparameter in der HCl-Phosgen-Trennung kann der Gehalt an gelöster HCl und gelösten inerten Gasen in dem in dem ersten Verfahrensschritt erzeugten flüssigen Strom so eingestellt werden, dass ein negativer Effekt auf die Reaktion nicht festgestellt werden kann.

Durch das erfindungsgemäße Verfahren in Kombination mit den erfindungsgemäßen Verfahrensbedingungen kann eine hohe Phosgenrückgewinnungsausbeute erreicht werden.

Somit wird durch den Einsatz des erfindungsgemäßen Verfahrens mit den darin geforderten Verfahrensbedingungen zum einen eine energetisch vorteilhafte Phosgenrückgewinnung und Phosgen-rückführung zu der Phosgenierreaktion erreicht und zum anderen die Sicherheit des Verfahrens erhöht. Des Weiteren ermöglicht das erfindungsgemäße Verfahren die Phosgenrückgewinnung und Phosgenrückführung mit einer geringeren Anzahl an Apparaten wodurch die Investitionskosten verringert werden können.

Die Erfindung betrifft ein Verfahren zur Herstellung von Isocyanaten durch Umsetzung von primären Aminen mit Phosgen im stöchiometrischen Überschuss in der Gasphase, bei dem
a) das Amin mit Phosgen in einem Reaktor oberhalb der Siedetemperatur des Amins umgesetzt wird, wobei ein das Isocyanat enthaltender flüssiger Strom und ein Gasstrom enthaltend HCl und Phosgen erhalten wird, und
b) der in Schritt a) erhaltene Gasstrom enthaltend HCl und Phosgen zunächst in einer Sequenz aus einem isotherm und einem adiabatisch geführten Absorptionsschritt in einen HCl enthaltenden Gasstrom und einen Phosgen enthaltenden flüssigen Strom aufgetrennt wird, und
c) der in Schritt b) erhaltene Phosgen enthaltende flüssige Strom dann in einen gasförmigen Strom enthaltend Phosgen und in einen flüssigen Strom aufgetrennt wird, wobei die Temperatur des aus Schritt b) erhaltenen Phosgen enthaltenden flüssigen Stroms vor Eintritt in Schritt c) erhöht wird, wobei die Temperaturerhöhung zwischen Austritt aus Schritt b) und Eintritt in Schritt c) zwischen 5 - 175°C liegt, und
d) der in Schritt c) erhaltene gasförmige Strom enthaltend Phosgen wieder in die Umsetzung in Schritt a) zurückgeführt wird, wobei
e) der Druck des in Schritt c) erhaltenen gasförmigen Stroms enthaltend Phosgen höher ist als der Druck des in Schritt b) erhaltenen Phosgen enthaltenden flüssigen Stroms.

### Gasphasenphosgenierung (Schritt a))

Die Phosgenierung von Aminen in der Gasphase durch Umsetzung mit Phosgen in Schritt a) ist allgemein aus dem Stand der Technik (z.B. EP-A-570 799, WO-A-2007/014936) bekannt.

Dabei werden bevorzugt primäre Amine eingesetzt. Bevorzugt werden primäre aromatische Amine eingesetzt, insbesondere bevorzugt primäre aromatische Diamine, die im Wesentlichen ohne Zersetzung in die Gasphase überführt werden können.

Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus.

Weiterhin sind besonders Amine, insbesondere Diamine, auf Basis von aliphatischen oder cycloaliphatischen Kohlenwasserstoffen mit 2 bis 18 Kohlenstoffatomen geeignet. Besonders gut geeignete Amine sind 1,6-Diamino-hexan, 1-Amino-3,3,5-trimethyl-5-aminomethylcyclohexan (IPDA) und 4,4'-Diaminodicyclohexylamin.

Die Ausgangsamine werden vor der Durchführung des erfindungsgemäßen Verfahrens in der Regel verdampft und auf 200°C bis 600°C, bevorzugt 200°C bis 500°C, besonders bevorzugt 250°C bis 450°C erhitzt und gegebenenfalls verdünnt mit einem Inertgas wie N₂, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt.

Die Verdampfung der Ausgangsamine kann in allen bekannten Verdampfungsapparaturen erfolgen, bevorzugt werden Verdampfungssysteme, bei denen ein kleiner Arbeitsinhalt mit einer hohen Umwälzleistung über einen Fallfilmverdampfer geführt wird, wobei zur Minimierung der thermischen Belastung der Ausgangsamine der Verdampfungsvorgang - wie oben ausgeführt- ggf. unter Zuspeisung von Inertgas und / oder Dämpfen eines inerten Lösungsmittels unterstützt werden kann. Alternativ kann die Verdampfung auch in speziellen Verdampfungsapparaturen erfolgen mit sehr kurzen Verweilzeiten wie sie zum Beispiel in EP 1 754 698 beschrieben sind.

Beim erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1:1 bis 20:1, bevorzugt 1,2:1 bis 5:1 vor. Auch das Phosgen wird auf Temperaturen von 200°C bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt.

Das erfindungsgemäße Verfahren wird so durchgeführt, dass die getrennt aufgeheizten Reaktionspartner über zumindest eine Mischeinrichtung in wenigstens einen Reaktionsraum eingeführt, vermischt und unter Beachtung geeigneter Reaktionszeiten unter bevorzugt adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbaminsäurechlorids, also beispielsweise Toluylendiaminsäurechlorid im Falle von TDA, erfolgt.

Die notwendige Verweilzeit zur Reaktion der Amingruppen mit dem Phosgen zu Isocyanat liegt zwischen 0,05 und 15 Sekunden, in Abhängigkeit von der Art des eingesetzten Amins, der Starttemperatur, der adiabaten Temperaturerhöhung im Reaktionsraum, dem molaren Verhältnis von eingesetztem Amin und Phosgen, einer etwaigen Verdünnung der Reaktionspartner mit Inertgasen sowie dem gewählten Reaktionsdruck.

Wird für das jeweilige System (eingesetztes Amin, Starttemperatur, adiabate Temperaturerhöhung, molares Verhältnis der Reaktanden, Verdünnungsgas, Reaktionsdruck) eine einmal ermittelte Mindestverweilzeit für die vollständige Reaktion um weniger als 20%, vorzugsweise weniger als 10% überschritten, kann die Bildung von Folge-Reaktionsprodukten wie Isocyanuraten und Carbodiimiden weitgehend vermieden werden.

Innerhalb dieses für chemische Reaktionen sehr engen Kontaktzeitspektrums muss sowohl die möglichst homogene Vermischung der Reaktionspartner als auch die weitere Reaktion erfolgen. Dabei erfolgt die weitere Reaktion bevorzugt ohne Rückvermischung, die eine Verbreiterung des Kontaktzeitraums und damit eine vermehrte Bildung von unerwünschten Neben- und Folgeprodukten bewirken würde.

Bei der praktischen Durchführung des Verfahrens kann eine Abweichung von der mittleren Kontaktzeit durch die notwendige Zeit der Vermischung der Reaktionspartner erfolgen. Die Methoden zur Durchführung kurzer Mischzeiten sind im Prinzip bekannt. Geeignet sind beispielsweise Mischaggregate oder Mischräume mit bewegten oder statischen Mischorganen oder Düsen. Bevorzugt ist der Einsatz statischer Mischer in Mischräumen, wie er z.B. in EP-A-1 362 847, EP-A-1 526 129 oder EP-A- 1 555 258 beschrieben ist. Bevorzugt werden in dem erfindungsgemäßen Verfahren die in den Absätzen (0008) bis (0014) und (0023) bis (0026) der EP-A-1 362 847, die in den Absätzen (0008) bis (0013) und (0022) bis (0026) der EP-A-1 526 129 oder die in den Absätzen (0007) und (0024) bis (0025) der EP-A-1 555 258 offenbarten Apparate eingesetzt.

Besonders bevorzugt kommen Reaktoren mit im Wesentlichen rotationssymmetrischen Reaktionsräumen zum Einsatz, bei denen die gasförmigen Edukte, ggf. verdünnt mit Inerten, dem zumindest einen Mischraum nach dem Strahlmischerprinzip (Chemie-Ing. Techn. 44 (1972) S. 1055, Abb. 10) zugeführt werden. Bevorzugt treten dabei die eingespeisten Stoffströme mit einem Geschwindigkeitsverhältnis von 2-20, besonders bevorzugt 3-15, ganz besonders bevorzugt 4-12, in den zumindest einen Mischraum der Reaktoren ein. Bevorzugt wird dabei dem zumindest einen Mischraum der Reaktoren das Amin, ggf. verdünnt mit Inerten, mit der höheren Strömungsgeschwindigkeit zugeführt.

Bevorzugt weisen weder der Reaktionsraum noch etwaige Mischaggregate oder Mischräume Heizflächen auf, die zu einer thermischen Belastung mit der Folge von Folgereaktionen wie Isocyanurat- oder Carbodiimidbildung Anlass geben können, oder Kühlflächen auf, die Anlass einer Kondensation mit der Folge von Ablagerungen geben können. Die Komponenten werden so, abgesehen von etwaigen Abstrahlungs- und Ableitungsverlusten, bevorzugt adiabat umgesetzt, dabei wird die adiabate Temperaturerhöhung im Mischaggregat und Reaktor bzw. Reaktor allein über die Temperaturen, Zusammensetzungen und relativen Dosierungen der Eduktströme sowie der Verweilzeit in den Mischaggregaten und den Reaktoren eingestellt. Möglich ist bei dem erfindungsgemäßen Verfahren auch eine nicht adiabatische Umsetzung der Komponenten.

Nach der erfolgten Phosgenierungsreaktion im Reaktionsraum wird das gasförmige Reaktionsgemisch, das bevorzugt wenigstens ein Isocyanat, Phosgen und Chlorwasserstoff umfasst, von dem gebildeten Isocyanat befreit. Dies kann beispielsweise dadurch erfolgen, indem das den Reaktionsraum kontinuierlich verlassende Gemisch, bevorzugt wenigstens ein Isocyanat, Phosgen und Chlorwasserstoff umfassend, nach Verlassen des Reaktionsraumes einer Kondensation in einem inerten Lösungsmittel unterzogen wird, wie sie bereits für andere Gasphasenphosgenierungen empfohlen wurde **(**EP-A-0 749 958**).**

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der eingesetzten Amine und des Phosgens zu den entsprechenden Isocyanaten ein oder mehrere geeignete Flüssigkeitsströme ("Quench-Flüssigkeiten") eingesprüht werden. Dadurch kann, wie in EP-A-1 403 248 beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Zone (Kühlzone) in eine Quenchstufe, wie sie z. B. in EP-A-1 403 248 offenbart wurde, integriert. In einer besonders bevorzugten Form kommen mehrere Kühlzonen zum Einsatz, erfolgen Integration und Betrieb dieser zumindest zwei Kühlzonen mit einer Quenchstufe. Dies ist hinsichtlich Aufbau und Betrieb in EP-A-1 935 875 offenbart.

Statt des integrierten Verbundes von der zumindest einen Kühlzone eines Reaktors mit einer Quenchstufe, wie sie in der EP-A-1 935 875 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

In einer bevorzugten Ausführungsform des erfindungsgemäßen Verfahrens beträgt die Durchsatzkapazität des eingesetzten Reaktors bei den erfindungsgemäß geforderten Reaktionsbedingungen > 1 t Amin / h und bevorzugt 2 - 50 t Amin / h. Besonders bevorzugt gelten diese Werte für Toluylendiamin, 1,6-Diamino-hexan und für Isophorondiamin. Unter Durchsatzkapazität ist dabei zu verstehen, dass in dem Reaktor die genannte Durchsatzkapazität an Amin pro h umgesetzt werden kann.

Unabhängig von der Art der gewählten Abkühlung wird die Temperatur der zumindest einen Abkühlzone bevorzugt so gewählt, dass sie einerseits oberhalb der Zersetzungstemperatur des dem Isocyanat entsprechenden Carbamoylchlorids liegt und andererseits das Isocyanat und gegebenenfalls das in dem Amindampfstrom und / oder Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und ggf. als Verdünnungsmittel mit verwendetes Inertgas die Kondensations- bzw. Quenchstufe weitestgehend nicht kondensiert bzw. gelöst durchlaufen. Zur selektiven Gewinnung des Isocyanats aus dem gasförmigen Reaktionsgemisch besonders gut geeignet sind bei einer Temperatur von 80 bis 200°C, vorzugsweise 80 bis 180 °C gehaltene Lösungsmittel wie beispielsweise Chlorbenzol und / oder Dichlorbenzol, oder in diesen Temperaturbereichen gehaltenes Isocyanat oder Mischungen des Isocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Isocyanates in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quencheflüssigkeit löst.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit.

Die Reindarstellung der Isocyanate erfolgt bevorzugt anschließend durch destillative Aufarbeitung der Lösungen bzw. Gemische aus der Kondensations- bzw. Quenchstufe.

Der aus Schritt a) erhaltene Gasstrom enthaltend zumindest HCl und Phosgen wird anschließend in Schritt b) in einen HCl enthaltenden Gasstrom und einen Phosgen enthaltenden flüssigen Strom aufgetrennt.

### HCl - Phosgen - Trennung (Schritt b))

Das den Schritt a) verlassende Gasgemisch enthaltend zumindest HCl und den nicht umgesetzten Phosgenüberschuss der Reaktion wird erfindungsgemäß in der HCl-Phosgen Trennung in Schritt b) in einen gasförmigen im wesentlichen HCl enthaltenden Strom und einen flüssigen Phosgen enthaltenden Strom aufgetrennt.

Das aus dem Schritt a) kommende und in die Auftrennung in Schritt b) eintretende Gasgemisch kann neben dem Reaktionskoppelprodukt HCl und dem nicht umgesetzten Phosgenüberschuss zudem gegebenenfalls inerte Gase und/oder Lösemittel und/oder Reaktionsnebenprodukte und/oder Spuren des Reaktionsproduktes enthalten. Als inerte Gase seien beispielsweise Stickstoff, Helium, Argon sowie überschüssiges CO aus der Phosgenherstellung und CO₂ erwähnt. Als Reaktionsnebenprodukte seien beispielsweise die Nebenprodukte aus der Phosgenherstellung wie zum Beispiel Tetrachlormethan, Trichlormethan, Monochlormethan, CO₂ und Methan erwähnt.

Das in die Auftrennung in Schritt b) eintretende Gasgemisch enthält in der Regel 1 bis 50 Gew.-% HCl, bevorzugt 3 - 40 Gew.-% HCl, insbesondere bevorzugt 5 - 35 Gew.-% HCl und ganz besonders bevorzugt 7,5 - 30 Gew.-% HCl, bezogen auf das Gewicht des Gasgemisches. Dieses Gasgemisch enthält in der Regel 5-90 Gew.-% Phosgen, bevorzugt 15 - 85 Gew.-% Phosgen, insbesondere bevorzugt 25 - 80 Gew.-% Phosgen und ganz besonders bevorzugt 40 - 75 Gew% Phosgen, bezogen auf das Gewicht des Gasgemisches. Der Gehalt an Lösemittel in dem Gasgemisch beträgt in der Regel 0,01 - 60 Gew.-%, bevorzugt 0,05 - 40 Gew.-% und insbesondere bevorzugt 0,1 - 10 Gew.-%, bezogen auf das Gewicht des Gasgemisches. Das Lösemittel kann dampfförmig oder auch flüssig vorliegen. Das Gasgemisch kann zudem inerte Gase in Summe von in der Regel 0 - 10 Gew.-%, bevorzugt 0,0001 - 8 Gew.-% und insbesondere bevorzugt 0,001 - 5 Gew.-%, bezogen auf das Gewicht des Gasgemisches, enthalten. Das Gasgemisch kann in der Regel 0 - 10 Gew.-%, bevorzugt 0,001 - 7,5 Gew.-% und insbesondere bevorzugt 0,05 - 5 Gew.-%, bezogen auf das Gewicht des Gasgemisches, an Reaktionsprodukt enthalten.

Alle in dieser Schrift angegebenen Zusammensetzungen beziehen sich auf Gewicht der jeweiligen Komponenten in Bezug auf das Gewicht des jeweiligen Gesamtstromes, sofern nicht in den entsprechenden Passagen eine andere Definition vorgenommen wird.

Die erfindungsgemäße Trennung des den Schritt a) verlassenden Gasstromes enthaltend HCl und den nicht umgesetzten Phosgenüberschuss der Reaktion kann durch verschiedene Ausführungsformen erfolgen. Erfindungsgemäß vorgesehen für diesen Verfahrensschritt ist eine Absorption in einem Lösemittel. Insbesondere wird die Absorption in einem Lösemittel durchgeführt, das auch für die Quenche verwendet wird. Insbesondere bevorzugt wird das gleiche in der Quenche eingesetzte Lösemittel verwendet.

Erfindungsgemäß wird Schritt b) durch Absorption in einer Sequenz aus zumindest 2 Absorptionsschritten gegebenenfalls in Kombination mit partiellen Kondensationsstufen, wobei zumindest ein Absorptionsschritt isotherm und zumindest ein Absorptionsschritt adiabatisch geführt wird, durchgeführt. Bevorzugt wird der erste Absorptionsschritt isotherm, der folgende Absorptionsschritt adiabatisch geführt. In der bevorzugten Ausführungsform erfolgt die Absorption in dem Lösemittel das für die Reaktion verwendet wird. In einer insbesondere bevorzugten Ausführungsform wird für den adiabatischen und isothermen Absorptionsschritt jeweils das gleiche auch in der Reaktion eingesetzte Lösemittel verwendet. Es ist weiterhin bevorzugt das die letzte Absorptionsstufe verlassende Gas durch Abkühlen mittels eines Wärmetauschers von verbleibenden Spuren an Phosgen und Lösemittel durch Kondensation weiter zu reinigen. In einer bevorzugten Ausführungsform erfolgt die isotherme und folgende adiabatische Absorption in einem Apparat, besonders bevorzugt erfolgt auch die Abkühlung des die Absorption verlassenden Gasstromes in dem gleichen Apparat. Dieses hat den Vorteil, dass dadurch die Anzahl der Flansche verringert wird, was zu einer Erhöhung der Sicherheit bei der Handhabung von Phosgen beiträgt. Es hat den weiteren Vorteil der Energieeinsparung, da durch die kompakte Bauweise in einem Apparat Energieverluste in den verbindenden Rohrleitungen minimiert werden.

In einer ganz besonders bevorzugten Ausführungsform wird das den Schritt a) verlassende Gasgemisch vor Eintritt in die Absorptionsstufe partiell kondensiert, wobei ein flüssiger und ein gasförmigen Strom erhalten wird. Bevorzugt wird diese Kondensation so durchgeführt, dass in dem flüssigen Strom Phosgen, gegebenenfalls Lösemittel und nur geringe Mengen gelöste HCl enthalten sind und dass in dem gasförmigen Strom HCl und gegebenenfalls Phosgen und Inerte enthalten sind. Der bei der partiellen Kondensation erhaltende gasförmige Strom wird der Absorptionstufe zugeführt. Bevorzugt erfolgt die Kondensationsstufe bei Temperaturen von -40 - 0°C, insbesondere bevorzugt bei Temperaturen von -20 - 0°C. Bevorzugt erfolgt die Kondensation in einem Rohrbündelwärmetauscher, ganz bevorzugt in einem vertikalen Rohrbündelwärmetauscher. Insbesondere bevorzugt wird der Apparat von oben nach unten durchströmt. Zur Verbesserung der Kondensationswirkung kann gegebenenfalls Lösemittel zugesetzt werden. Bevorzugt hat das Lösemittel eine Temperatur von unterhalb 10°C, insbesondere bevorzugt von weniger als 0°C. Das Lösemittel kann phosgenfrei oder phosgenhaltig sein.

In einer weiteren ganz besonders bevorzugten Ausführungsform werden die Dämpfe aus der Kondensationsstufe anschließend im Gegenstrom mit dem in der Reaktion verwendeten Lösemittel geführt. Dabei wird das Phosgen gegebenenfalls gemeinsam mit Spuren HCl und/oder inerten Gasen und/oder Reaktionsnebenprodukten in dem Lösemittel absorbiert. Bevorzugt steigt das Gas von unten nach oben durch die Absorptionsstufen, bevorzugt läuft das Lösemittel mit der Schwerkraft von oben nach unten durch die Absorptionsstufen. In einer besonders bevorzugten Ausführungsform wird der in der Kondensationsstufe erhaltende flüssige Strom mit dem aus dem Absorptionsstufen ablaufenden flüssigen Strömen im Sumpf des Apparates kombiniert.

In einer weiteren bevorzugten Ausführungsform wird für den adiabatischen Absorptionsschritt Lösemittel mit einer Temperatur von -40 - 0°C, bevorzugt -20 bis -10°C verwendet. Es ist weiterhin bevorzugt, dass dieses Lösemittel weniger als 1000 ppm Phosgen enthält, bevorzugt weniger als 500 ppm, insbesondere bevorzugt weniger als 250 ppm. In einer insbesondere bevorzugten Ausführungsform wird für die isotherme Absorption das aus dem adiabatischen Absorptionsschritt bereits mit Phosgen beladene Lösemittel verwendet. Es ist jedoch auch denkbar für den isothermen Absorptionsschritt gegebenenfalls zusätzlich oder ausschließlich andere Phosgen enthaltende Lösemittelströme zu verwenden, wie sie zum Beispiel in der Destillation von Phosgenieranlagen anfallen. In einer bevorzugten Ausführungsform beträgt der adiabatische Temperaturanstieg 0,1 - 20°C, insbesondere 2 - 5°C.

Die Menge des im Absorptionsschritt aufgegebenen Lösemittels beträgt das 0,1 - 5fache, bevorzugt das 0,15 - 3 fache der Masse bezogen auf die Masse des in den Verfahrenschritt a) eintretenden Gasgemisches. Durch Wahl der aufgegebenen Menge, der Temperatur und der Zusammensetzung des verwendeten Lösemittels ggfs. in Kombination mit der Einstellung der Verfahrensparameter wie z.B. Druck und Temperatur in der HCl-Phosgen-Trennung kann die Qualität des aus der Absorptionsstufe in Schritt b) austretenden Gasstroms als auch die Zusammensetzung des Schritt b) verlassenden flüssigen Phosgen enthaltenden Stroms beeinflusst werden.

Der isotherme Absorptionsschritt wird bevorzugt in einem Rohrbündelwärmetauscher, insbesondere in vertikaler Installation, durchgeführt. Dabei wird die freigesetzte Absorptionswärme in der Waschflüssigkeit direkt während der Entstehung auf die Wärmetauscheroberfläche übertragen und abgeführt. Bevorzugt wird der Apparat auf der Mantelseite gekühlt, bevorzugt hat das Kühlmittel eine Eintrittstemperatur von -40 bis 0°C, insbesondere bevorzugt -25 bis -10°C.Die Anzahl der Rohre kann breit variieren und ist nur durch die technische Fertigbarkeit begrenzt. Denkbar sind Apparate mit 100 bis 6000 Rohren, mit einer Rohrlänge von 1 bis 10 m, bevorzugt 3 bis 8 m. Der Rohrdurchmesser kann zwischen 10 bis 200 mm variieren, bevorzugt liegt der Rohrdurchmesser im Bereich 20 - 100 mm. Zur Vergrößerung der Kontaktfläche können die Rohre gegebenenfalls mit einem Füllmaterial ganz oder teilweise gefüllt sein. Hierzu sind verschiedene Packungen oder Füllkörpersysteme dem Fachmann bekannt.

Der den isothermen Absorptionsschritt verlassende Gasstrom enthält bevorzugt im wesentlichen HCl und inerte Gase sowie Restmengen noch nicht absorbiertes Phosgen. Die Gastemperatur liegt in der Regel zwischen -20°C - 10°C, bevorzugt zwischen -20 - 0°C. In der Regel enthält der den isothermen Absorptionsschritt verlassende Gasstrom noch bis zu 5 Gew% Phosgen, bevorzugt bis zu 4 Gew% Phosgen und insbesondere bevorzugt bis zu 3 Gew%, bezogen jeweils auf das Gewicht des Gasgemisches. In der Regel enthält der den isothermen Absorptionsschritt verlassende Gasstrom noch mehr als 0,05 Gew% Phosgen, bevorzugt mehr als 0,1 Gew% und insbesondere mehr als 0,15 Gew% Phosgen, bezogen jeweils auf das Gewicht des Gasgemisches.

Der sich bevorzugt an den isothermen Absorptionsschritt anschließende adiabatische Absorptionsschritt wird bevorzugt in einer Kolonne durchgeführt. Diese kann mit Böden, Packungen oder Füllkörpern ausgerüstet sein. Bevorzugt hat der adiabatische Absorptionsschritt 1 bis 50 theoretische Trennstufen, insbesondere bevorzugt 2 bis 40 theoretische Trennstufen. Die Absorptionskolonne kann eine Länge von 2 - 25 Metern haben, bevorzugt sind 3 - 18 Meter. Der Kolonnendurchmesser ist nur durch die technische Fertigbarkeit liminiert, in der Regel liegt der Kolonnendurchmesser im Bereich 250 - 5000 mm, bevorzugt im Bereich 500 - 4000 mm.

In einer bevorzugten Ausführungsform beträgt der gesamte Druckverlust über die isotherme und adiabatische Absorptionsstufe weniger als 250 mbar, bevorzugt weniger als 200 mbar und insbesondere bevorzugt weniger als 150 mbar. Dieses meint, dass der Druck des Gases am Eintritt in die isotherme Absorptionsstufe nicht mehr als 250 mbar höher, bevorzugt nicht mehr als 200 mbar höher, insbesondere bevorzugt nicht mehr als 150 mbar höher ist als der Druck des aus der adiabatischen Absorptionsstufe austretenden Gases.

Die aus dem oder den Absorptionsstufen und Kondensationsstufen ablaufenden flüssigen Ströme haben bevorzugt nur noch eine sehr geringe Beladung mit gelöster HCl und / oder gelösten inerten Gasen und können ohne weitere Reinigung in die erfindungsgemäßen zweiten Verfahrensschritt der Phosgengaserzeugung geleitet werden. Bevorzugt werden die aus der Kondensationstufe und der Absorptionsstufe ablaufenden Ströme kombiniert und als ein gemeinsamer Strom in den zweiten Verfahrensschritt der Phosgengaserzeugung in Schritt c) geleitet.

Die beschriebenen Verfahrensalternativen zur Durchführung des Schrittes b) liefern alle einen gasförmigen Strom und einen flüssigen Strom. Der HCl enthaltende Gasstrom hat eine ausreichende Reinheit und kann im Allgemeinen ohne weitere Reinigung weiter verarbeitet werden.

Der Schritt b) verlassende Gasstrom enthaltend HCl enthält im wesentlichen HCl sowie gegebenenfalls Spuren von Phosgen. Neben HCl kann der Strom zudem noch inerte Gase und / oder Lösemittel sowie Spuren an Reaktionsnebenprodukten enthalten. Der Strom enthält 80 - 100 Gew.-%, bevorzugt 90 - 100 Gew.-% und insbesondere bevorzugt 95 - 100 Gew.-% HCl, bezogen auf das Gewicht des Gasstroms enthaltend HCl. Dieser Gasstrom enthält maximal 0,8 Gew.-% Phosgen, bevorzugt maximal 0,4 Gew.-% und insbesondere bevorzugt maximal 0,2 Gew.-% Phosgen, bezogen auf das Gewicht des Gasstroms enthaltend HCl. Zur energetischen Optimierung kann es bevorzugt sein mindestens 1 Gew-ppm Phosgen, bevorzugt mindestens 5 Gew-ppm Phosgen bezogen auf das Gewicht des Gasstroms enthaltend HCl in dem Schritt b) verlassenden Gasstrom zuzulassen. Zudem kann dieser Strom 0 - 10 Gew.-%, bevorzugt 0,01 - 7,5 Gew.-% und insbesondere bevorzugt 0,05 - 5 Gew.-% inerte Gase, bezogen auf das Gewicht des Gasstroms enthaltend HCl enthalten. Weiterhin kann dieser Strom 0 - 1 Gew.-%, bevorzugt 0,001 - 0,5 Gew.-% und insbesondere bevorzugt 0,05 - 0,2 Gew.-% Lösemittel, bezogen auf das Gewicht des Gasstroms enthaltend HCl enthalten. Der mögliche Gehalt an Reaktionsnebenprodukten beträgt in der Regel max 1 Gew.-%, bevorzugt maximal 0,5 Gew.-% und insbesondere bevorzugt 0,25 Gew.-%, bezogen auf das Gewicht des Gasstroms enthaltend HCl.

Der Schritt b) verlassende Gasstrom steht in der Regel am Austritt aus dem Verfahrensschritt unter einem Druck von 1 bis 4 bar absolut, bevorzugt von 1,01 bis 3 bar absolut und insbesondere bevorzugt 1,02 bis 2 bar absolut. Der aus Schritt b) erhaltene Gasstrom hat in der Regel am Austritt aus dem Verfahrensschritt eine Temperatur von -40 bis 30°C, bevorzugt - 20 bis 20 und insbesondere bevorzugt - 15 bis 10°C. Als Austritt aus dem Verfahrensschritt wird der Gasaustrittstutzen des letzten zu diesem Verfahrensschritt gehörigen Apparates verstanden.

Der Schritt b) verlassende flüssige Phosgen enthaltende Strom kann in der Regel neben Phosgen noch Lösemittel und / oder gelöste HCl und / oder gelöste Inerte sowie gegebenenfalls gelöste Reaktionsnebenprodukte enthalten. Diesen Strom enthält 30 - 90 Gew.-%, bevorzugt 35 - 85 Gew.-%, insbesondere bevorzugt 38 - 75 Gew.-% und ganz besonders bevorzugt 40 - 70 Gew.-% Phosgen, bezogen auf das Gewicht des flüssigen Phosgen enthaltenden Stroms. Zudem kann dieser Strom 10 - 70 Gew.-% Lösemittel, bevorzugt 15 - 65 Gew.-% und insbesondere bevorzugt 25 - 60 Gew.-%, bezogen auf das Gewicht des flüssigen Phosgen enthaltenden Stroms enthalten. Weiterhin kann dieser flüssige Strom 0 - 5 Gew.-% gelöste HCl enthalten, bevorzugt 0.1 - 3.5 Gew% und insbesondere bevorzugt maximal 0,5 - 2,5 Gew.-% gelöste HCl, bezogen auf das Gewicht des flüssigen Phosgen enthaltenden Stroms. Weiterhin kann dieser flüssige Strom gegebenenfalls gelöste inerte Gase von in Summe maximal 1 Gew.-%, bevorzugt maximal 0,5 Gew.-% und insbesondere bevorzugt 0,1 Gew.-%, bezogen auf das Gewicht des flüssigen Phosgen enthaltenden Stroms enthalten. Dieser Strom enthält in Summe 1 Gew-ppm gelöste inerte Gase, bevorzugt 10 Gew-ppm, bezogen auf das Gewicht des flüssigen Phosgen enthaltenden Stroms. Der Gehalt an gegebenenfalls vorhandenen Reaktionsnebenprodukten beträgt in der Regel 0 - 5 Gew.-%, bevorzugt 0.001 - 3 Gew.-% und insbesondere bevorzugt 0.05 - 2,5 Gew.-%, bezogen auf das Gewicht des flüssigen Phosgen enthaltenden Stroms.

Der aus dem ersten Verfahrensschritt austretende flüssige Phosgen enthaltende Strom hat in der Regel eine Temperatur von - 40 bis 20°C, bevorzugt -25 bis 15°C und insbesondere bevorzugt -20 bis 10°C und ganz besonders bevorzugt -15 bis 8°C. Dabei steht dieser Strom in der Regel bei Austritt aus dem Verfahrensschritt unter einem Druck von 1 bis 4 bar absolut, bevorzugt 1,01 bis 3 bar absolut und insbesondere bevorzugt von 1,02 bis 2 bar absolut. Als Austritt aus dem Verfahrens-schritt für den flüssigen Phosgen enthaltenden Strom wird der Flüssigkeitsaustrittsstutzen aus dem oder den zu dieser Verfahrensstufe gehörigen Apparaten verstanden. Dabei wird der dort gemessene Druck um den hydrostatischen Druck der Flüssigkeitssäule in dem oder den Apparaten bereinigt.

Der erfindungsgemäße geringe Gehalt an gelöster HCl und / oder gelösten inerten Gases in dem in Schritt b) erzeugten flüssigen Phosgen enthaltenden Strom wirkt sich energetisch vorteilhaft auf die Phosgengaserzeugung in Schritt c) aus, da dadurch die in Schritt c) zu erzeugende Gesamtgasmenge geringer ist und somit dafür ein geringerer Energieaufwand in Schritt c) notwendig ist. Zudem führt der erfindungsgemäße geringe Gehalt an gelöster HCl und / oder gelösten inerten Gases in dem in Schritt b) erzeugten flüssigen Phosgen enthaltenden Strom nicht zu einer intolerablen Inertgasbelastung in den nachfolgenden Apparaten entlang des Phosgengasweges.

### Phosgen - Gaserzeugung (Schritt c))

Der aus der HCl-Phosgen Trennung in Schritt b) erhaltene flüssige Phosgen enthaltende Strom wird erfindungsgemäß in die Phosgengaserzeugung in Schritt c) geleitet. Da das die Phosgengaserzeugung in Schritt c) verlassende Gas erfindungsgemäß unter einem höheren Druck steht als der die HCl-Phosgen Trennung in Schritt b) verlassende flüssige Strom enthaltend Phosgen, muss der von Schritt b) nach Schritt c) geleitete flüssige Strom eine Druckdifferenz überwinden. Dieses kann durch geschicktes Aufstellen der Apparate durch Höhendifferenz mittels Gravitation oder durch Anlegen eines Gasdruckes erfolgen. Bevorzugt erfolgt es über eine Pumpe. Die Überführung des flüssigen Stroms von Schritt b) nach Schritt c) kann kontinuierlich oder diskontinuierlich, bevorzugt kontinuierlich, erfolgen.

Erfindungsgemäß wird die Phosgengaserzeugung in Schritt c) so durchgeführt, dass der aus Schritt b) erhaltene flüssige Phosgen enthaltende Strom in Schritt c) in einen gasförmigen Strom und in einen flüssigen Strom aufgetrennt wird. Dieses kann bevorzugt durch Destillation oder partielle Verdampfung erfolgen.

In einer bevorzugten Ausführungsform wird die Phosgengaserzeugung in Schritt c) so durchgeführt, dass der Phosgen enthaltende flüssige Strom aus Schritt b) in einen im wesentlichen Phosgen und Inerte enthaltenden gasförmigen Strom und in einen flüssigen Strom aufgetrennt wird. Erfindungsgemäß ist dabei der Druck in diesem Verfahrensschritt (Schritt c)) höher als der Druck des in der HCl / Phosgen - Trennung (Schritt b)) erhaltenden flüssigen Stromes.

In einer bevorzugten Ausführungsform erfolgt die Phosgengaserzeugung in Schritt c) in einer Destillationskolonne mit 1 - 80 theoretischen Trennstufen, bevorzugt 2 - 45 theoretischen Trennstufen. Die Kolonne kann einen Abtriebsteil und / oder Verstärkerteil enthalten, bevorzugt enthält die Kolonne sowohl einen Abtriebs- als auch einen Verstärkerteil. Der Abtriebsteil hat bevorzugt 1 - 40 theoretische Trennstufen, besonders bevorzugt 1 - 20 theoretische Trennstufen. Der Verstärkerteil hat bevorzugt 1 - 40 theoretische Trennstufen, besonders bevorzugt 1 - 20 theoretische Trennstufen. Die Destillationskolonne kann mit Böden, Packungen oder Füllkörpern ausgerüstet sein, bevorzugt sind Böden oder Packungen. Geeignete Böden oder Packungen sind dem Fachmann bekannt, erwähnt seien zum Beispiel ohne Beschränkung strukturierte Blech- oder Gewebepackungen, Glocken-, Sieb oder Ventilböden.

Die Kolonne wird in der Regel bei einer Sumpftemperatur von 100 bis 250°C, bevorzugt von 120 bis 230°C und insbesondere bevorzugt von 140 - 220°C betrieben.

Der Differenzdruck der Destillationskolonne ist in der Regel geringer als 400 mbar, bevorzugt geringer als 300 mbar und insbesondere geringer als 200 mbar. Unter Differenzdruck ist dabei die Druckdifferenz zwischen dem Kopf- und dem Sumpfdruck der Kolonne zu verstehen.

In einer bevorzugten Ausführungsform ist die Kolonne mit einem Kopfkondensator versehen, insbesondere bevorzugt ist der Kopfkondensator in die Kolonne eingesteckt. Der Kopfkondensator wird in der Regel bei einer Kühlmitteleintrittstemperatur von - 40 bis 20°C betrieben, bevorzugt bei - 30 bis 10°C und insbesondere bevorzugt bei - 25 bis 0°C. In einer besonders bevorzugten Ausführungsform ist der Differenzdruck des Gases über den Kopfkondensator kleiner als 150 mbar, insbesondere bevorzugt kleiner als 100 mbar. Das durch den Kopfkondensator erzeugte Kondensat kann ganz oder teilweise auf die Kolonne zurückgeführt und / oder entnommen werden, bevorzugt wird das Kondensat vollständig auf die Kolonne zurückgeführt.

Die Energiezufuhr im Sumpf der Kolonne kann durch jeden denkbaren Verdampfer erfolgen, wie zum Beispiel Naturumlaufverdampfer, Kletterverdampfer und Fallfilmverdampfer. Insbesondere bevorzugt sind Fallfilmverdampfer.

In einer bevorzugten Ausführungsform erfolgt die Aufgabe des aus Schritt b) erhaltenen flüssigen Stroms in die Mitte der Kolonne, bevorzugt erfolgt die Aufgabe zwischen den Verstärker- und Abtriebsteil der Kolonne.

In einer besonders bevorzugten Ausführungsform hat die Kolonne zudem eine Kopfaufgabe, bevorzugt befindet sich diese Aufgabeposition oberhalb des Verstärkerteils. In einer besonders bevorzugten Form ist dieses eine flüssige Aufgabeposition. In einer ganz besonders bevorzugten Ausführungsform wird durch diese Aufgabeposition flüssiges Phosgen zugeführt.

Das gegebenenfalls am Kopf der Kolonne zugeführte flüssige Phosgen hat in der Regel eine Temperatur von - 30 bis 10°C, bevorzugt - 20 bis 0°C. In der Regel enthält dieser Strom im wesentlichen Phosgen, d.h. der Phosgengehalt liegt zwischen 95 - 100 Gew.-%, bevorzugt liegt der Phosgengehalt zwischen 98 - 100 Gew.-%, bezogen auf das Gewicht dieses Stromes. Durch die Aufgabe von flüssigem Phosgen auf den Kopf der Kolonne kann der energetische Bedarf verringert werden.

In einer weiteren Ausführungsform kann die Kolonne zudem eine Aufgabeposition für einen gasförmigen Strom enthalten. Diese Aufgabeposition befindet sich bevorzugt unterhalb oder oberhalb des Verstärkerteils oder auch unterhalb des Abtriebsteils.

In einer weiteren möglichen Ausführungsform wird die Phosgengaserzeugung in Schritt c) so durchgeführt, dass der Phosgen enthaltende flüssige Strom aus Schritt b) in einen Phosgen und ggf. Inerte enthaltenden gasförmigen Strom und in einen flüssigen Strom durch partielle Verdampfung aufgetrennt wird. Erfindungsgemäß ist dabei der Druck in der Phosgengaserzeugung in Schritt c) höher als der Druck des in der HCl / Phosgen - Trennung (Schritt b)) erhaltenden flüssigen Stromes.

Dazu wird der aus Schritt b) erhaltene flüssige Strom in einen Verdampfer geführt. Der Verdampfer wird mit einem externen Heizmittel beheizt. Die Sumpftemperatur des Verdampfers liegt im Bereich von 30 bis 250°C, bevorzugt von 70 bis 230°C und insbesondere bevorzugt im Bereich von 100 - 220°C.

Neben dem flüssigen Strom aus Schritt b) kann zudem ein weiterer flüssiger Phosgenstrom in den Verdampfer eingeleitet werden. Dieser weitere flüssige Phosgenstrom hat in der Regel eine Temperatur von -30 bis 10°C, bevorzugt -20 bis 0°C. In der Regel enthält der Strom im wesentlichen Phosgen, d.h. der Phosgengehalt liegt zwischen 95 - 100 Gew.-%, bevorzugt liegt der Phosgengehalt zwischen 98 -100 Gew.-%, bezogen auf das Gewicht dieses Stromes.

In dem Verdampfer wird die Flüssigkeit partiell verdampft, d.h. es erfolgt eine kontinuierliche oder diskontinuierliche flüssige Ausschleusung aus dem Verdampfer, bevorzugt erfolgt die Ausschleusung kontinuierlich.

Es ist weiterhin möglich, die Phosgengaserzeugung in den verschiedenen Ausführungsformen durch z.B. Einblasung von inerten Gasen wie z.B. Stickstoff zu unterstützen.

Der in der Phosgengaserzeugung in Schritt c) erhaltene gasförmige Strom enthält im wesentlichen Phosgen. Neben Phosgen kann dieser Strom noch inerte Gase und / oder Lösemittel und / oder Reaktionsnebenprodukte und / oder HCl enthalten. In der Regel enthält dieser Gasstrom 80 - 100 Gew.-%, bevorzugt 85 - 99.9 Gew.-%, insbesondere bevorzugt 90 - 99.8 Gew.-% und ganz besonders bevorzugt 92 - 99.7 Gew.-% Phosgen, bezogen auf das Gewicht des Gasstroms. Zudem kann dieser Strom bis zu 20 Gew.-% dampfförmiges Lösemittel, bevorzugt bis zu 15 Gew.-% und insbesondere bevorzugt zwischen 5 Gew.-ppm und bis zu 10 Gew.-% Lösemittel, bezogen auf das Gewicht des Gasstroms, enthalten. Zur Optimierung des Energieeinsatzes ist es sinnvoll, einen gewissen Gehalt an Lösemittel in diesem gasförmigen Strom zuzulassen. Dieser liegt in der Regel bei mindestens 5 Gew-ppm, bevorzugt bei mindestens 10 Gew-ppm und insbesondere bevorzugt bei mindestens 25 Gew.ppm, bezogen auf das Gewicht des Gasstroms. Dieser Strom kann in der Regel in der Summe maximal 1 Gew.-% inerte Gase, bevorzugt maximal 0.5 Gew.-% und insbesondere bevorzugt 0,1 Gew.-% , bezogen auf das Gewicht des Gasstroms enthalten. Weiterhin kann dieser Strom maximal 5 Gew.-% HCl enthalten, bevorzugt maximal 4,0 Gew.-% und insbesondere bevorzugt maximal 3,5 Gew.-% HCl, bezogen auf das Gewicht des Gasstroms. Der Gehalt an gegebenenfalls vorhandenen Reaktionsnebenprodukten beträgt in der Regel bis zu 5 Gew.-%, bevorzugt bis zu 4 Gew.-% und insbesondere bevorzugt bis zu 2,5 Gew.-%, bezogen auf das Gewicht des Gasstroms.

Der in der Phosgengaserzeugung erhaltende gasförmige Strom enthaltend Phosgen hat in der Regel am Austritt aus diesem Verfahrensschritt eine Temperatur von -10 - 100°C, bevorzugt 0 - 80°C und insbesondere bevorzugt 5 - 70°C. Der Druck des erhaltenden gasförmigen Stroms beträgt in der Regel am Austritt aus diesem Verfahrensschritt 1,05 bis 6 bar absolut, bevorzugt 1,3 bis 6 bar und insbesondere bevorzugt 1,6 bis 6 bar. Als Austritt aus der Verfahrensstufe wird der Gasaustrittstutzen aus dem oder den Apparaten, in dem Schritt c) durchgeführt wird, verstanden.

Erfindungsgemäß ist der Druck des in Schritt c) erhaltenen gasförmigen Stroms enthaltend Phosgen stets höher als der Druck des in Schritt b) erhaltenen Phosgen enthaltenden flüssigen Stroms. Dabei bezieht sich die geforderte Druckdifferenz auf den Druck des gasförmigen Stroms enthaltend Phosgen beim Austritt aus dem oder den Apparaten, in dem Schritt c) durchgeführt wird und den Druck des in Schritt b) erhaltenen Phosgen enthaltenden flüssigen Stroms beim Austritt aus dem oder den Apparaten in denen Schritt b) durchgeführt wird bereinigt um den hydrostatischen Druck der Flüssigkeitssäule in dem oder den Apparaten. In der Regel und bevorzugt beträgt diese Druckdifferenz mindestens 50 mbar, besonders bevorzugt mindestens 100 mbar und ganz besonders bevorzugt mindestens 250 mbar und übersteigt im Allgemeinen nicht 100 bar. Die Druckdifferenz kann bevorzugt durch eine Pumpe für den in Schritt b) erhaltenen Phosgen enthaltenden flüssigen Strom aufgebracht werden, die bevorzugt zwischen den Apparaten, in denen die Schritte b) und c) durchgeführt werden, angeordnet ist.

Dabei ist zudem bevorzugt der Druck des in Schritt c) erhaltenen gasförmigen Stroms enthaltend Phosgen stets höher als der Druck des in Schritt b) erhaltenen HCl enthaltenden Gasstromes. Dabei beträgt die Druckdifferenz zwischen dem Austritt des gasförmigen Stromes aus Schritt c) und dem Austritt des HCl enthaltenden Gasstromes aus Schritt b) bevorzugt mindestens 50 mbar, bevorzugt mindestens 100 mbar und insbesondere bevorzugt mindestens 250 mbar. Die Druckdifferenz zwischen Schritt b) und Schritt c) übersteigt im Allgemeinen nicht 100 bar.

Bevorzugt kann damit in Schritt d) durch diese Druckführung der die Phosgengaserzeugung in Schritt c) verlassende gasförmige Strom enthaltend Phosgen ohne Einsatz eines Druckerhöhungselements im Gasweg in die Umsetzung in Schritt a) geleitet werden. Durch den Verzicht eines Druckerhöhungselements im Gasweg wird die Verfahrenssicherheit verbessert, da auf die sicherheitstechnisch schwierige gasdichte Abdichtung von rotierenden Teilen verzichtet werden kann. Bevorzugt kann der in der Reaktion in Schritt a) gebildete Gasstrom dann ebenfalls ganz oder teilweise ohne den Einsatz von Druckerhöhungselementen bis in die HCl-Phosgen-Trennung in Schritt b) geführt werden. Insbesondere bevorzugt wird somit gasseitig komplett auf den Einsatz von Druckerhöhungselementen für den Phosgenkreislauf (Schritte a) bis d)) verzichtet.

In der Phosgengaserzeugung in Schritt c) wird ein flüssiger Strom erhalten, der im Wesentlichen aus Lösemittel besteht. Neben diesem kann dieser Strom auch noch Reaktionsnebenprodukte erhalten. Weiterhin kann der Strom noch gewisse Mengen an Phosgen enthalten. Dieser flüssige Strom enthält in der Regel 80 - 100 Gew.-% Lösemittel, bevorzugt 85 - 99,9 Gew.-%, insbesondere bevorzugt 90 - 99,8 Gew.-% und ganz besonders bevorzugt 95 - 99,7 Gew.-%, bezogen auf das Gewicht des flüssigen Stroms.

Zudem kann dieser Strom bis zu 20 Gew.-% gelöstes Phosgen, bevorzugt bis zu 15 Gew.-% und insbesondere bevorzugt bis zu 10 Gew.-% und ganz besonders bevorzugt bis zu 7 Gew.-% Phosgen, bezogen auf das Gewicht des flüssigen Stroms, enthalten. Zur Optimierung des Energieeinsatzes ist es sinnvoll einen gewissen Gehalt an Phosgen in diesem flüssigen Strom zuzulassen. Dieser liegt in der Regel bei mindestens 1 Gew-ppm, bevorzugt bei mindestens 3 Gew-ppm und insbesondere bevorzugt bei mindestens 8 Gew.ppm, bezogen auf das Gewicht des flüssigen Stroms. Dieser Strom ist in der Regel in der Summe mit maximal 0,5 Gew% gelösten inerten Gasen beladen, bevorzugt maximal 0,1 Gew% und insbesondere bevorzugt 0,05 Gew.-%, bezogen auf das Gewicht des flüssigen Stroms. Weiterhin kann dieser Strom maximal 1 Gew.-% HCl enthalten, bevorzugt maximal 0,1 Gew.-% und insbesondere bevorzugt maximal 0,05 Gew.-% HCl, bezogen auf das Gewicht des flüssigen Stroms. Der Gehalt an gegebenenfalls vorhandenen Reaktionsnebenprodukten beträgt in der Regel bis zu 5 Gew.-%, bevorzugt bis zu 4 Gew.-% und insbesondere bevorzugt bis zu 2,5 Gew.-% , bezogen auf das Gewicht des flüssigen Stroms.

Zur Optimierung des Energieverbrauches der erfindungsgemäßen Verfahrensschritte ist es vorgesehen, den in der HCl / Phosgen -Trennung in Schritt b) erhaltenen Phosgen enthaltenden flüssigen Strom indirekt, d.h. nach weiterer prozesstechnischer Veränderung in die Phosgengaserzeugung in Schritt c) zu leiten.

Unter prozesstechnischer Veränderung wird im Sinne dieser Schrift die Veränderung der Zusammensetzung, des Druckes oder der Temperatur verstanden.

Der aus Schritt b) erhaltene flüssige Strom wird indirekt in die Phosgengaserzeugung in Schritt c) geleitet, wobei die Temperatur des flüssigen Stroms erhöht wird. Dabei liegt die Temperaturerhöhung des Stromes zwischen Austritt aus Schritt b) und Eintritt in Schritt c) zwischen 5 - 175°C.

Insbesondere bevorzugt wird die Temperatur durch Austausch mit zumindest einem anderen flüssigen Stoffstoffstrom der Anlage erhöht. Bevorzugt findet dieser Austausch in einem Wärmetauscher wie zum Beispiel einem Rohrbündelwärmetauscher oder Plattenwärmetauscher, bevorzugt einem Rohrbündelwärmetauscher statt.

Zur Minimierung des apparatetechnischen Aufwandes und damit zur Minimierung der Investitionskosten ist es sinnvoll, die Gesamtanzahl der Apparate in denen diese prozesstechnische Veränderung des Stromes erfolgt, zu begrenzen. Im Allgemeinen wird in nicht mehr als 15 Apparaten in Reihe diese verfahrenstechnische Veränderung durchgeführt, bevorzugt in nicht mehr als 10 Apparate in Reihe und besonders bevorzugt in nicht mehr als 8 Apparaten in Reihe. Durch diese Beschränkung wird die Anzahl der verbindenden Rohrleitungen und Flansche und damit die Gefahr einer Leckage verringert. Dieses erhöht somit die Sicherheit des Verfahrens.

Zur Optimierung des Energieverbrauches der erfindungsgemäßen Verfahrensschritte ist es gegebenenfalls sinnvoll den in Schritt c) erhaltenen flüssigen Strom vor der weiteren Verwendung in dem Gesamtprozess prozesstechnisch zu verändern.

Dieser Strom kann gegebenenfalls ganz oder teilweise als Lösemittel in der HCl-Phosgen - Trennung in Schritt b) eingesetzt werden. Dieses ist insbesondere vorteilhaft, um leicht siedende Reaktionsprodukte gemeinsam mit dem in Schritt b) erhaltenen Gasstrom aus dem Verfahren zu entfernen.

Durch das erfindungsgemäße Verfahren ist es möglich eine hohe Phosgenrückgewinnungsausbeute zu erzielen. Unter Phosgenrückgewinnungsausbeute wird dabei der Anteil des Phosgens verstanden, der durch den erfindungsgemäßen Schritt b) aus dem den Reaktor verlassenden Gasgemisch enthaltend zumindest HCl und den nicht umgesetzten Phosgenüberschuss der Reaktion abgetrennt, und über den im Schritt c) erhaltenden gasförmigen Strom wieder in der Umsetzung nach Schritt a) zugeführt wird.

Zur Berechnung der Phosgenrückgewinnungsausbeute wird der Quotient in Prozent aus der Phosgenmenge des in den Verfahrenschritt b) eintretenden Gasstromes und der Phosgenmenge des aus dem Verfahrensschritt c) austretenden Gasstromes gebildet, wobei gegebenenfalls zugeführtes Frisch-Phosgen abgezogen wird.

Im Allgemeinen beträgt die Phosgenrückgewinnungsausbeute mehr als 90%, insbesondere mehr als 93%, bevorzugt mehr als 95% und insbesondere bevorzugt mehr als 98%.

Das erfindungsgemäße Verfahren bietet die vorteilhafte Möglichkeit, im gesamten Phosgengasraum auf den Einsatz von Druckerhöhungselementen für gasförmiges Phosgen zu verzichten.

Durch den bevorzugten Verzicht auf diese Aggregate wird die Sicherheit der Produktionsanlage erhöht, da diese Aggregate oftmals eine Wellenabdichtung besitzen, deren Abdichtung gerade bei dem Einsatz von gasförmigen Phosgen technisch aufwendig und sicherheitstechnisch problematisch ist. Desweiteren ist der Einsatz von Druckerhöhungselementen oftmals energieintensiv, so dass durch den Verzicht auf diese Elemente der Energiebedarf der Anlage verbessert ist.

Unter Druckerhöhungselementen im Sinne der vorliegenden Schrift ist ein technisches Aggregat zu verstehen, das den Druck eines gasförmigen Stromes erhöht. Dieses bedeutet, dass der Druck des Gases im Eintritt in das Aggregat geringer ist als der Druck des Gases bei Austritt aus dem Aggregat. Denkbare Druckerhöhungselemente sind zum Beispiel Kompressoren, Verdichter oder Jets.

Unter Phosgengasraum im Sinne der vorliegenden Erfindung ist der Gasraum zu verstehen, in dem Phosgen in nennenswerter Menge gasförmig vorhanden ist. Unter nennenswerter Menge Phosgen ist zu verstehen, dass der Gehalt an Phosgen im Gasraum >lGew.-% beträgt. Im Besonderen ist unter gesamten Phosgengasraum der Bereich beginnend bei dem Verfahrensabschnitt der Phosgengaserzeugung (Schritt c), Phosgen-Rückführung (Schritt d), der Phosgenierung (Schritt a) und der HCl-Phosgen - Trennung (Schritt b) sowie der Phosgenerzeugung zu verstehen.

### Phosgen-Rückführung (Schritt d))

Der im erfindungsgemäßen Verfahren erhaltene gasförmige Strom aus Schritt c) wird vollständig oder teilweise in die Umsetzung nach Schritt a) zurückgeführt. Bevorzugt wird dieser Strom vollständig in die Umsetzung nach Schritt a) zurückgeführt. Insbesondere ist es nicht notwendig, einen Teil des in Schritt c) erhaltenden gasförmigen Stromes wieder in die HCl-Phosgen-Trennung (Schritt b) zu führen.

Es ist denkbar den in Schritt c) erhaltenen Gasstrom geregelt oder ungeregelt auf die Reaktoren zu verteilen, bevorzugt erfolgt die Verteilung geregelt. Die Anzahl der Reaktoren, in die der aus Schritt c) erhaltende gasförmige Strom, gegebenenfalls kombiniert mit einem in der Phosgenerzeugung erhaltenden gasförmigen Strom, geleitet wird, muss zumindest eins sein. Denkbar ist jedoch auch eine größere Anzahl von Reaktoren, bevorzugt ist die Anzahl kleiner 20. Denkbar ist auch, dass im Falle des Einsatzes von mehr als einem Reaktor ein oder mehrere Reaktoren ausschließlich Frisch-Phosgen erhalten, während die verbleibenden Reaktoren ausschließlich mit dem aus der Phosgengaserzeugung in Schritt c) erhaltenen Gasstrom versorgt werden.

Es ist ebenfalls möglich, dass der aus mehreren Reaktoren erhaltene Gasstrom aus Schritt a) kombiniert in die HCl-Phosgen - Trennung in Schritt b) geleitet wird. Bevorzugt sind bis zu 20 Reaktoren mit ihren jeweiligen den Schritt a) verlassenden Gasströmen an eine gemeinsame HCl-Phosgen - Trennung in Schritt b) angebunden. Dazu können die Gasströme gemeinsam, getrennt und teilweise vereint in den Verfahrensschritt b) geleitet werden. Das in dem Verfahrenschritt a) hergestellte Isocyanat kann gleich oder verschieden sein.

Das für die Phosgenierung notwendige Frisch-Phosgen, d.h. das in der Regel durch Umsetzung von Chlor mit Kohlenstoffmonooxid erzeugte Phosgen, kann auf verschiedene Arten in das erfindungsgemäße Verfahren eingebracht werden. Unter Frisch-Phosgen im Sinne der vorliegenden Anmeldung wird solches Phosgen verstanden, welches nicht unmittelbar aus dem erfindungsgemäßen Verfahren stammt. Bevorzugt handelt es sich dabei um solches Phosgen, welches nach der Synthese von Phosgen, zumeist aus Chlor und Kohlenstoffmonoxid, keine Reaktionsstufe mit einer Phosgenumsetzung von mehr als 5% des Umsatzes der in der Phosgensynthese hergestellten Phosgens durchläuft.

Zum einen ist es möglich das Frisch-Phosgen gasförmig zu verwenden. Dieses gasförmige Phosgen kann mit dem aus der Phosgengaserzeugung aus Schritt c) erhaltenen Gasstrom vermischt und als gemeinsamer Strom zu den Phosgenierreaktoren in Schritt a) geleitet werden. Dieses ist zum Beispiel im Stand der Technik aus EP-A-2028179 bekannt. Das gasförmige Frisch-Phosgen kann gegebenenfalls kombiniert mit einem in der Phosgenerzeugung in Schritt c) erhaltenen gasförmigen Strom gemeinsam oder getrennt auf die Reaktoren der Phosgenierreaktion in Schritt a) geleitet werden.

Weiterhin ist es möglich, den in der Phosgenerzeugung erzeugten gasförmigen Frisch-Phosgenstrom gemeinsam mit dem den Reaktor verlassenden Strom in die HCl-Phosgen - Trennung in Schritt b) zu leiten. Dieses ist nach Lehre von EP-A-1 849 767 vorteilhaft um Verunreinigungen aus der HCl zu strippen und um einen besonders reinen HCl Strom zu erhalten.

Weiterhin ist es möglich, das Frisch-Phosgen gasförmig in die Phosgengaserzeugung in Schritt c) zu leiten. Dieses ist vorteilhaft, da durch die Gaseinleitung die Verdampfung des flüssigen Stromes in diesem Verfahrenschritt durch den Strippeffekt erleichtert wird.

Sofern das Frisch-Phosgen gasförmig in das erfindungsgemäße Verfahren eingebracht wird, ist der Druck in der Phosgenerzeugung höher als der Druck des in Schritt c) erhaltenden gasförmigen Stromes. In der Regel ist der Druck in der Phosgenerzeugung um mindestens 50 mbar höher, bevorzugt mindestens 80 mbar höher und insbesondere bevorzugt mindestens 100 mbar höher als der Druck in Schritt c). Die Druckdifferenz zwischen der Phosgenerzeugung und des in Schritt c) erhaltenden gasförmigen Stromes beträgt in der Regel nicht mehr als 100 bar.

Zum anderen ist es möglich das Frisch-Phosgen zunächst zu verflüssigen und dadurch von inerten Gasen und Nebenprodukten der Phosgenherstellung weitestgehend zu reinigen. In diesem Fall ist es möglich, das so erhaltende flüssige Phosgen in die HCl-Phosgen - Trennung in Schritt b) zu leiten. Es ist jedoch auf möglich, dieses flüssige Phosgen in die Phosgengaserzeugung in Schritt c) zu führen. Es ist weiterhin möglich in einer separaten Apparatur das so erzeugte flüssige Phosgen zu verdampfen, wodurch ein gasförmiger Phosgenstrom erhalten wird, der entsprechend den im vorherigen Absatz beschriebenen Möglichkeiten in das erfindungsgemäße Verfahren eingebracht werden kann.

Sofern das Frisch-Phosgen zunächst verflüssigt wird, ist der Druck in der Phosgenerzeugung unabhängig von dem Druck in Schritt c), d.h. der Druck in der Phosgenerzeugung kann höher, gleich oder niedriger sein als der Druck in Schritt c), in der Regel ist der Druck in der Phosgenerzeugung (Frisch-Phosgen) höher als der Druck in Schritt c). Wesentlich ist lediglich, dass das verflüssigte Frisch-Phosgen dem erfindungsgemäßen Verfahren zugeführt wird. Dieses kann durch geschicktes Aufstellen der Apparate durch Höhendifferenz mittels Gravitation oder durch Anlegen eines Gasdruckes erfolgen. Bevorzugt erfolgt es über eine Pumpe. Die Überführung des verflüssigten Frisch-Phosgens in das erfindungsgemäße Verfahren kann kontinuierlich oder diskontinuierlich, bevorzugt kontinuierlich, erfolgen.

Bevorzugt kann durch die beschriebene Anbindung der Phosgenerzeugung an das erfindungsgemäße Verfahren das Frisch-Phosgen ohne Einsatz eines Druckerhöhungselements im Gasweg in das erfindungsgemäße Verfahren geleitet werden. Durch den Verzicht eines Druckerhöhungselements im Gasweg wird die Verfahrenssicherheit verbessert, da auf die sicherheitstechnisch schwierige gasdichte Abdichtung von rotierenden Teilen verzichtet werden kann.

Erfindungsgemäß ist der Druck des in Schritt c) erhaltenen gasförmigen Stroms enthaltend Phosgen stets höher als der Druck des in Schritt b) erhaltenen Phosgen enthaltenden flüssigen Stroms. Dabei ist zudem bevorzugt der Druck des in Schritt c) erhaltenden Gases stets höher als der Druck des in Schritt b) erhaltenden Gases. Weiterhin ist der Druck in dem Schritt a) geringer als der Druck des in Schritt c) erhaltenden Gases. Weiterhin ist der Druck in Schritt a) höher als der Druck des in Schritt b) erhaltenden Gases. Somit ergibt sich für das Gesamtverfahren bestehend aus Schritt a) (Gasphasenphosgenierung), Schritt b) (HCl-Phosgen-Trennung), Schritt c) (Phosgengaserzeugung) das der Druck in Schritt c) am höchsten und in Schritt b) am niedrigsten ist, während der Druck in Schritt a) zwischen den Drücken in Schritten c) und b) liegt. Durch dieses erfindungsgemäße Verfahren kann im gesamten Phosgengasraum auf den Einsatz von Druckerhöhungselementen für gasförmiges Phosgen verzichtet und somit die Anlagensicherheit verbessert werden.

Die in der Regel vorhandene Druckdifferenz zwischen dem Austritt des gasförmigen Stromes aus Schritt c) und dem Austritt des HCl enthaltenden Gasstromes aus Schritt b) von mindestens 50 mbar, bevorzugt von mindestens 100 mbar und insbesondere von bevorzugt mindestens 250 mbar stellt sicher, dass das Gas ohne zusätzliches Druckerhöhungselement von der Phosgengaserzeugung (Schritt c) über die Gasphasenphosgenierung (Schritt a) zur HCl-Phosgen-Trennung (Schritt b) strömt. Es ist nicht erfindungswesentlich wie sich die gesamte Druckdifferenz zwischen Schritt c) und Schritt b) aufteilt, sofern die Druckdifferenz zwischen Schritt c) und Schritt a) mindestens 20 mbar und die Druckdifferenz zwischen Schritt a) und Schritt b) mindestens 20 mbar beträgt. Diese Druckdifferenz stellt eine ausreichend schnelle Gasgeschwindigkeit sicher, so dass auf den Einsatz eines Druckerhöhungselementes in dem Phosgengasraum verzichtet werden kann.

### Beispiele:

Angaben in ppm sind im Rahmen dieser Anmeldung als auf das Gewicht bezogen zu verstehen (Gew.-ppm). Die Angaben in mbara bedeuten den absoluten Druck in mbar.

### Beispiel 1 (erfindungsgemäß):

In einem Rohrreaktor mit nachgeschalteter Isocyanatkondensationsstufe wird durch Vermischung in einer Düse ein Gemisch bestehend aus gasförmigen 2,4- und 2,6-Toluylendiamin sowie Stickstoff als Inertgas zusammen mit einem gasförmigen Phosgenstrom zur Reaktion gebracht. Der Reaktordruck beträgt 1600 mbara, die Reaktionstemperatur ca. 450°C. Dabei wird ein flüssiger Strom enthaltend 2,4- und 2,6-Toluylendiisocyanat sowie ein gasförmiger Strom enthaltend Phosgen und HCl erhalten. Der isocyanathaltige Strom wurde destillativ zum reinen Toluylendiisocyanat gereinigt.

Der gasförmige Strom HCl und Phosgen enthaltende Strom wird durch eine dreistufige Prozessführung in einen HCl Gasstrom und einen Phosgen enthaltenden flüssigen Strom aufgetrennt. Zunächst wird der Strom partiell kondensiert, dann wird das verbleibende Gas durch einen isothermen und anschließend durch einen adiabaten Absorptionsschritt geleitet. Zudem wird kaltes Lösemittel mit einer Temperatur von -11°C am Kopf des adiabaten Absorptionsschrittes aufgegeben und fließt im Gegenstrom mit dem Gas durch die Absorptionsschritte. Am Kopf der Absorptionskolonne wird HCl Gas mit einem Phosgengehalt von weniger als 50 Gew.-ppm entnommen, im Sumpf der Absorptionskolonne wird eine Lösung bestehend aus ODB und Phosgen unter einem Druck von ca. 1400 mbara auf Höhe des Flüssigkeitsspiegels erhalten. Der Druck des HCl Gases am Kopf der Absorptionskolonne beträgt 1300 mbara.

Die so erhaltende Phosgenlösung wird mit einer Pumpe in die Phosgengaserzeugung, die in Form einer Desorptionskolonne ausgestaltet ist, gefördert. Der Zulauf erfolgt zwischen den Abtriebs-und Verstärkerteil der Kolonne. Am Kopf der Kolonne wird ein gasförmiger Strom enthaltend 94,4 Gew% Phosgen und 4,4 Gew% HCl mit einem Druck von 1800 mbara entnommen. Dieser Strom wird gasförmig mit einem Phosgengasstrom (Frisch Phosgen) aus der Phosgenerzeugung gemischt und zur Düse des Rohrreaktors geführt.

In dem gesamten Phosgengasraum wird kein Druckerhöhungselement eingesetzt, die Phosgenrückgewinnungsausbeute beträgt 98,3%.

### Beispiel 2 (erfindungsgemäß):

In einer zweistrassigen Anlage zur Herstellung von Diisocyanaten durch Phosgenierung in der Gasphase wird in der ersten Phosgenierstrasse in einem Rohrreaktor mit nachgeschalteter Isocyanatkondensationsstufe durch Vermischung in einer Düse ein Gemisch bestehend aus gasförmigen Isophorondiamin sowie Stickstoff als Inertgas zusammen mit einem gasförmigen Phosgenstrom, der direkt aus der Phosgenerzeugung eingeleitet wird, zur Reaktion gebracht. Der Reaktordruck beträgt 1300 mbara, die Reaktionstemperatur ca. 400°C. Das Reaktionsgemisch wird durch Entzug von Wärme mit oder ohne Zugabe von Lösungsmittel stark abgekühlt, wobei eine flüssige Phase enthaltend im wesentlichen Isophorondiisocyanat und eine gasförmige, isocyanatfreie Phase enthaltend im wesentlichen den Phosgenüberschuss sowie das Nebenprodukt HCl am Austritt aus der Kühlzone erhalten wird. Der isocyanathaltige Strom wird destillativ zum reinen Isophorondiisocyanat gereinigt.

In einer zweistrassigen Anlage zur Herstellung von Diisocyanaten durch Phosgenierung in der Gasphase wird in der zweiten Phosgenierstrasse in einem Rohrreaktor mit nachgeschalteter Isocyanatkondensationsstufe durch Vermischung in einer Düse ein Gemisch bestehend aus gasförmigen 1,6-Diaminohexan sowie Stickstoff als Inertgas zusammen mit einem gasförmigen Phosgenstrom, der aus einem Gemisch eines Phosgengasstroms (Frisch Phosgen) aus der Phosgenerzeugung und eines Phosgengasstroms aus einer Desorptionskolonne besteht, eingeleitet wird, zur Reaktion gebracht. Der Reaktordruck beträgt 1300 mbara, die Reaktionstemperatur ca. 430°C. Das Reaktionsgemisch wird durch Entzug von Wärme mit oder ohne Zugabe von Lösungsmittel stark abgekühlt, wobei eine flüssige Phase enthaltend im wesentlichen 1,6-Hexandiisocyant und eine gasförmige, isocyanatfreie Phase enthaltend im wesentlichen den Phosgenüberschuss sowie das Nebenprodukt HCl am Austritt aus der Kühlzone erhalten wird. Der isocyanathaltige Strom wird destillativ zum reinen 1,6-Diisocyanatohexan gereinigt.

Die gasförmigen HCl und Phosgen enthaltenden Ströme aus beiden Phosgenierstrassen werden zusammengeführt und gemeinsam durch eine dreistufige Prozessführung in einen HCl Gasstrom und einen Phosgen enthaltenden flüssigen Strom aufgetrennt. Zunächst wird der Strom partiell kondensiert, dann wird das verbleibende Gas durch einen isothermen und anschließend durch einen adiabaten Absorptionsschritt geleitet. Zudem wird kaltes Lösemittel mit einer Temperatur von -11°C am Kopf des adiabaten Absorptionsschrittes aufgegeben und fließt im Gegenstrom mit dem Gas durch die Absorptionsschritte. Am Kopf der Absorptionskolonne wird HCl Gas mit einem Phosgengehalt von weniger als 0,5 Gew.-% entnommen, im Sumpf der Absorptionskolonne wird eine Lösung bestehend aus Monochlorbenzol (MCB) und Phosgen unter einem Druck von ca. 1150 mbara auf Höhe des Flüssigkeitsspiegels erhalten. Der Druck des HCl Gases am Kopf der Absorptionskolonne beträgt 1080 mbara.

Die so erhaltende Phosgenlösung wird mit einer Pumpe in die Phosgengaserzeugung, die in Form einer Desorptionskolonne ausgestaltet ist, gefördert. Der Zulauf erfolgt zwischen den Abtriebs-und Verstärkerteil der Kolonne. Am Kopf der Kolonne wird ein gasförmiger Strom enthaltend 94,4 Gew% Phosgen und 4,4 Gew% HCl mit einem Druck von 1400 mbara entnommen. Dieses Strom wird gasförmig mit einem Phosgengasstrom (Frisch Phosgen) aus der Phosgenerzeugung gemischt und zur Düse des Rohrreaktors der zweiten Phosgenierstrasse geführt.

In dem gesamten Phosgengasraum wird kein Druckerhöhungselement eingesetzt, die Phosgenrückgewinnungsausbeute beträgt 98,5%.

### Beispiel 3 (erfindungsgemäß):

In einem Rohrreaktor entsprechend Beispiel 1 wird ein flüssiger Strom enthaltend 2,4- und 2,6-Toluylendiissocyanat sowie ein gasförmiger Strom enthaltend Phosgen und HCl erhalten. Die Auftrennung des HCl und Phosgen haltigen Gasgemisches erfolgt wie in Beispiel 1 beschrieben. Der Druck des die Absorptionskolonne verlassenden HCl Gases am Gasaustrittsstutzen beträgt 1300 mbara, der Druck der im Sumpf des Apparates vorhandenen Phosgenlösung gemessen am Flüssigkeitsaustrittsstutzen abzüglich der durch die hydrostatische Flüssigkeitssäule verursachten Druckes beträgt 1400 mbara.

Die erhaltende Phosgenlösung wird mit einer Pumpe in die Phosgengaserzeugung, die in Form einer Desorptionskolonne ausgestaltet ist, gefördert. Der Zulauf erfolgt zwischen den Abtriebs-und Verstärkerteil der Kolonne. Zudem wird flüssiges Frisch Phosgen oberhalb des Verstärkerteils der Kolonne zugeführt. Am Kopf der Kolonne wird ein gasförmiger Strom enthaltend 97,6 Gew% Phosgen und 2,4 Gew% HCl mit einem Druck von 1900 mbara entnommen und wieder der Reaktion zugeführt.

In dem gesamten Phosgengasraum wird kein Druckerhöhungselement eingesetzt, die Phosgenrückgewinnungsausbeute beträgt 99%.

### Beispiel 4 (erfindungsgemäß):

In einem Rohrreaktor entsprechend Beispiel 1 wird ein flüssiger Strom enthaltend 2,4- und 2,6-Toluylendiissocyant sowie ein gasförmiger Strom enthaltend Phosgen und HCl erhalten. Die Auftrennung des HCl und Phosgen-haltigen Gasgemisches erfolgt wie in Beispiel 1 beschrieben. Der Druck des die Absorptionskolonne verlassenden HCl Gases am Gasaustrittsstutzen beträgt 1300 mbara, der Druck der im Sumpf des Apparates vorhandenen Phosgenlösung gemessen am Flüssigkeitsaustrittsstutzen abzüglich der durch die hydrostatische Flüssigkeitssäule verursachten Druckes beträgt 1400 mbara.

Die so erhaltende Phosgenlösung wird in eine Desorptionskolonne geleitet. Die Desorptionskolonne ist nur mit einem Verstärkerteil mit 20 theoretischen Stufen ausgerüstet. Der Feed zu der Kolonne erfolgt unterhalb des Verstärkerteils. Am Kopf der Kolonne wird gasförmiges Phosgen mit einem Druck von 2000 mbara abgenommen und wieder der Reaktion zugeführt. Im Sumpf der Kolonne wird bei einer Temperatur von ca. 115°C ODB entnommen, das 6,7 Gew% Phosgen enthält.

In dem gesamten Phosgengasraum wird kein Druckerhöhungselement eingesetzt, die Phosgenrückgewinnungsausbeute beträgt 95,6%.

### Beispiel 5 (erfindungsgemäß):

In einem Rohrreaktor entsprechend Beispiel 1 wird ein flüssiger Strom enthaltend 2,4- und 2,6-Toluylendiissocyanat sowie ein gasförmiger Strom enthaltend Phosgen und HCl erhalten. Die Auftrennung des HCl und Phosgen haltigen Gasgemisches erfolgt wie in Beispiel 1 beschrieben. Der Druck des die Absorptionskolonne verlassenden HCl Gases am Gasaustrittsstutzen beträgt 1300 mbara, der Druck der im Sumpf des Apparates vorhandenen Phosgenlösung gemessen am Flüssigkeitsaustrittsstutzen abzüglich der durch die hydrostatische Flüssigkeitssäule verursachten Druckes beträgt 1400 mbara.

Die so erhaltende Phosgenlösung wird mit einer Temperatur unterhalb von 10°C auf den Kopf einer Strippkolonne mit 20 theoretischen Stufen gepumpt. Am Kopf der Strippkolonne wird bei einem Druck von 2000 mbara gasförmiges Phosgen entnommen, das ca. 0,2 Gew% Lösemittel enthält. Im Sumpf der Strippkolonne Lösemittel mit ca 100 Gew.ppm Phosgen entnommen.

In dem gesamten Phosgengasraum wird kein Druckerhöhungselement eingesetzt, die Phosgenrückgewinnungsausbeute beträgt 99%.

### Beispiel 6 (erfindungsgemäß):

In einem Rohrreaktor mit nachgeschalteter Isocyanatkondensationsstufe wird durch Vermischung in einer Düse ein Gemisch bestehend aus gasförmigen 1,6-Diaminohexan sowie Stickstoff als Inertgas zusammen mit einem gasförmigen Phosgenstrom, der direkt aus der Phosgenerzeugung eingeleitet wird, zur Reaktion gebracht. Der Reaktordruck beträgt 1450 mbara, die Reaktionstemperatur ca. 450°C. Das Reaktionsgemisch wird durch Entzug von Wärme mit oder ohne Zugabe von Lösungsmittel stark abgekühlt, wobei eine flüssige Phase enthaltend im wesentlichen 1,6-Diisocyanatohexan und eine gasförmige, isocyanatfreie Phase enthaltend im wesentlichen den Phosgenüberschuss sowie das Nebenprodukt HCl am Austritt aus der Kühlzone erhalten wird. Der isocyanathaltige Strom wird destillativ zum reinen 1,6-Diisocyanatohexan gereinigt.

Der gasförmige HCl und Phosgen enthaltende Strom wird durch eine dreistufige Prozessführung in einen HCl Gasstrom und einen Phosgen enthaltenden flüssigen Strom aufgetrennt. Zunächst wird der Strom partiell kondensiert, dann wird das verbleibende Gas durch einen isothermen und anschließend durch einen adiabaten Absorptionsschritt geleitet. Zudem wird kaltes Lösemittel mit einer Temperatur von -11°C am Kopf des adiabaten Absorptionsschrittes aufgegeben und fließt im Gegenstrom mit dem Gas durch die Absorptionsschritte. Am Kopf der Absorptionskolonne wird HCl Gas mit einem Phosgengehalt von weniger als 0,5 Gew.-% entnommen, im Sumpf der Absorptionskolonne wird eine Lösung bestehend aus Monochlorbenzol (MCB) und Phosgen unter einem Druck von ca. 1250 mbara auf Höhe des Flüssigkeitsspiegels erhalten. Der Druck des HCl Gases am Kopf der Absorptionskolonne beträgt 1200 mbara.

Die so erhaltende Phosgenlösung wird mit einer Pumpe in die Phosgengaserzeugung, die in Form einer Desorptionskolonne ausgestaltet ist, gefördert. Der Zulauf erfolgt zwischen den Abtriebs-und Verstärkerteil der Kolonne. Am Kopf der Kolonne wird ein gasförmiger Strom enthaltend 93,3 Gew% Phosgen und 6,7 Gew% HCl mit einem Druck von 1800 mbara entnommen. Dieser Strom wird gasförmig mit einem Phosgengasstrom (Frisch Phosgen) aus der Phosgenerzeugung gemischt und zur Düse des Rohrreaktors geführt.

In dem gesamten Phosgengasraum wird kein Druckerhöhungselement eingesetzt, die Phosgenrückgewinnungsausbeute beträgt 98,5%.

## Patentansprüche

1. Verfahren zur Herstellung von Isocyanaten durch Umsetzung von primären Aminen mit Phosgen im stöchiometrischen Überschuss in der Gasphase, bei dem
a) das Amin mit Phosgen in einem Reaktor oberhalb der Siedetemperatur des Amins umgesetzt wird, wobei ein das Isocyanat enthaltender flüssiger Strom und ein Gasstrom enthaltend HCl und Phosgen erhalten wird, und
b) der in Schritt a) erhaltene Gasstrom enthaltend HCl und Phosgen zunächst in einer Sequenz aus einem isotherm und einem adiabatisch geführten Absorptionsschritt in einen HCl enthaltenden Gasstrom und einen Phosgen enthaltenden flüssigen Strom aufgetrennt wird, und
c) der in Schritt b) erhaltene Phosgen enthaltende flüssige Strom dann in einen gasförmigen Strom enthaltend Phosgen und in einen flüssigen Strom aufgetrennt wird, wobei die Temperatur des aus Schritt b) erhaltenen Phosgen enthaltenden flüssigen Stroms vor Eintritt in Schritt c) erhöht wird, wobei die Temperaturerhöhung zwischen Austritt aus Schritt b) und Eintritt in Schritt c) zwischen 5 - 175°C liegt, und
d) der in Schritt c) erhaltene gasförmige Strom enthaltend Phosgen wieder in die Umsetzung in Schritt a) zurückgeführt wird, wobei
e) der Druck des in Schritt c) erhaltenen gasförmigen Stroms enthaltend Phosgen höher ist als der Druck des in Schritt b) erhaltenen Phosgen enthaltenden flüssigen Stroms.

2. Verfahren nach Anspruch 1, bei dem in Schritt d) die Rückführung des in Schritt c) erhaltenen gasförmigen Stroms enthaltend Phosgen ohne den Einsatz von Druckerhöhungselementen durchgeführt wird.

3. Verfahren nach Anspruch 1, bei dem in Schritt c) ein gasförmiger Strom enthaltend Phosgen erhalten wird, der 80 - 100 Gew.-% an Phosgen, bezogen auf das Gewicht des gasförmigen Stroms, enthält.

4. Verfahren nach Anspruch 1, bei dem in Schritt b) ein Phosgen enthaltender flüssiger Strom erhalten wird, der 30 - 90 Gew.-% an Phosgen, bezogen auf das Gewicht des flüssigen Stroms, enthält.

5. Verfahren nach Anspruch 1, bei dem die Druckdifferenz in Schritt e) mindestens 50 mbar beträgt.

6. Verfahren nach Anspruch 1, bei dem der in Schritt a) gebildete Gasstrom enthaltend HCl und Phosgen ohne den Einsatz von Druckerhöhungselementen in die Trennung in Schritt b) geführt wird.

7. Verfahren nach Anspruch 1, bei dem der in Schritt b) erhaltene Phosgen enthaltende flüssige Strom mit einer Pumpe in Schritt c) gefördert wird.

## Claims

1. Process for the preparation of isocyanates by reacting primary amines with a stoichiometric excess of phosgene in the gas phase, wherein
a) the amine is reacted with phosgene in a reactor above the boiling point of the amine, where a liquid stream containing the isocyanate and a gas stream containing HCl and phosgene are obtained, and
b) the gas stream containing HCl and phosgene obtained in step a) is first separated, in a sequence consisting of an isothermal and an adiabatic absorption step, into a gas stream containing HCl and a liquid stream containing phosgene, and
c) the liquid stream containing phosgene obtained in step b) is then separated into a gas stream containing phosgene and into a liquid stream, where the temperature of the liquid stream containing phosgene obtained from step b) is raised before entering step c), where the increase in temperature between exiting step b) and entering step c) is between 5-175°C, and
d) the gas stream containing phosgene obtained in step c) is recycled into the reaction in step a), where
e) the pressure of the gas stream containing phosgene obtained in step c) is higher than the pressure of the liquid stream containing phosgene obtained in step b).

2. Process according to Claim 1 wherein, in step d), the recycling of the gas stream containing phosgene obtained in step c) is carried out without the use of pressure-raising elements.

3. Process according to Claim 1 wherein a gas stream containing phosgene is obtained in step c), which contains 80 - 100 wt.% of phosgene, based on the weight of the gas stream.

4. Process according to Claim 1 wherein a liquid stream containing phosgene is obtained in step b), which contains 30 - 90 wt.% of phosgene, based on the weight of the liquid stream.

5. Process according to Claim 1 wherein the pressure difference in step e) is at least 50 mbar.

6. Process according to Claim 1 wherein the gas stream containing HCl and phosgene formed in step a) is passed on to the separation in step b) without the use of pressure-raising elements.

7. Process according to Claim 1 wherein the liquid stream containing phosgene obtained in step b) is pumped into step c).

## Revendications

1. Procédé de fabrication d'isocyanates par mise en réaction d'aminés primaires avec du phosgène en excès stoechiométrique dans la phase gazeuse, selon lequel
a) l'amine est mise en réaction avec le phosgène dans un réacteur au-dessus de la température d'ébullition de l'amine, un courant liquide contenant l'isocyanate et un courant gazeux contenant HCl et le phosgène étant obtenus, et
b) le courant gazeux contenant HCl et le phosgène obtenu à l'étape a) est tout d'abord séparé dans une séquence d'une étape d'absorption réalisée sous forme isotherme et d'une étape d'absorption réalisée sous forme adiabatique en un courant gazeux contenant HCl et un courant liquide contenant du phosgène, et
c) le courant liquide contenant du phosgène obtenu à l'étape b) est ensuite séparé en un courant gazeux contenant du phosgène et en un courant liquide, la température du courant liquide contenant du phosgène obtenu à l'étape b) étant augmentée avant l'entrée dans l'étape c), l'élévation de température entre la sortie de l'étape b) et l'entrée dans l'étape c) étant comprise entre 5 et 175 °C, et
d) le courant gazeux contenant du phosgène obtenu à l'étape c) est recyclé dans la réaction à l'étape a),
e) la pression du courant gazeux contenant du phosgène obtenu à l'étape c) étant plus élevée que la pression du courant liquide contenant du phosgène obtenu à l'étape b).

2. Procédé selon la revendication 1, selon lequel, à l'étape d), le recyclage du courant gazeux contenant du phosgène obtenu à l'étape c) est réalisé sans utiliser d'éléments d'élévation de la pression.

3. Procédé selon la revendication 1, selon lequel, à l'étape c), un courant gazeux contenant du phosgène est obtenu, qui contient 80 à 100 % en poids de phosgène, par rapport au poids du courant gazeux.

4. Procédé selon la revendication 1, selon lequel, à l'étape b), un courant liquide contenant du phosgène est obtenu, qui contient 30 à 90 % en poids de phosgène, par rapport au poids du courant liquide.

5. Procédé selon la revendication 1, selon lequel la différence de pression à l'étape e) est d'au moins 50 mbar.

6. Procédé selon la revendication 1, selon lequel le courant gazeux contenant HCl et le phosgène formé à l'étape a) est conduit dans la séparation à l'étape b) sans utiliser d'éléments d'élévation de la pression.

7. Procédé selon la revendication 1, selon lequel le courant liquide contenant du phosgène obtenu à l'étape b) est transporté dans l'étape c) avec une pompe.
